(19) Europäisches Patentamt
European Patent Office
Office européen des brevets

(11) **EP 2 174 592 B1**

(12) **EUROPEAN PATENT SPECIFICATION**

(45) Date of publication and mention
of the grant of the patent:
**22.05.2013 Bulletin 2013/21**

(21) Application number: **08790424.9**

(22) Date of filing: **07.08.2008**

(51) Int Cl.:
*A61B 5/151* [(2006.01)]        *A61B 5/157* [(2006.01)]
*A61B 5/15* [(2006.01)]

(86) International application number:
**PCT/JP2008/002167**

(87) International publication number:
**WO 2009/019888 (12.02.2009 Gazette 2009/07)**

(54) **PIERCING DEVICE, BLOOD TESTING DEVICE, AND PIERCING METHOD**

DURCHSTECHVORRICHTUNG, BLUTTESTGERÄT UND DURCHSTECHVERFAHREN

DISPOSITIF DE PERÇAGE, DISPOSITIF DE TEST DU SANG ET PROCÉDÉ DE PERÇAGE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **07.08.2007 JP 2007205284**

(43) Date of publication of application:
**14.04.2010 Bulletin 2010/15**

(73) Proprietor: **Panasonic Corporation
Kadoma-shi
Osaka 571-8501 (JP)**

(72) Inventors:
• **MATSUMURA, Keisuke
  Osaka-shi, Osaka 540-6207 (JP)**
• **MATSUMOTO, Toshiki
  Osaka-shi, Osaka 540-6207 (JP)**

(74) Representative: **Grünecker, Kinkeldey,
Stockmair & Schwanhäusser
Leopoldstrasse 4
80802 München (DE)**

(56) References cited:
**JP-A- 2005 160 871     US-A1- 2004 267 300**

**Description**

Technical Field

The present invention relates to a puncturing apparatus that punctures skin and so forth, a blood test apparatus and a puncturing method. More specifically, the present invention relates to a puncturing apparatus that samples blood used for blood test, a blood test apparatus and a puncturing method.

Diabetes patients need to measure their blood sugar level on a regular basis and inject insulin based on the measured blood sugar level to maintain a normal blood sugar level. To maintain this normal blood sugar level, diabetes patients need to measure the blood sugar level on a regular basis. Therefore, patients puncture the skin of their fingers and so forth by using a blood test apparatus, sample a small amount of blood exuding from the skin and analyze the components, such as blood sugar level, based on the sampled blood.

Conventionally, the blood test apparatus disclosed in Patent Document 1 and Patent Document 2 have been known.

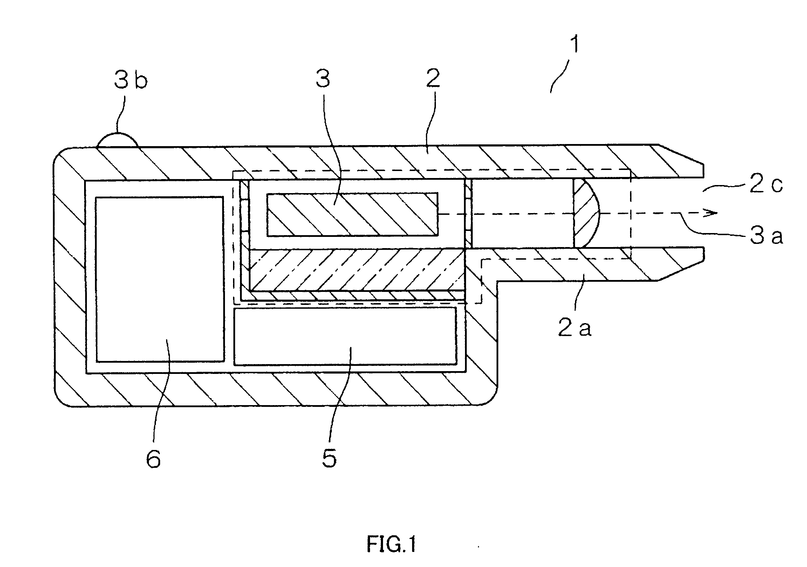

FIG.1 is a cross sectional view showing the configuration of the laser puncturing apparatus described in Patent Document 1.

In FIG.1, laser puncturing apparatus 1 has a configuration including: housing 2; tubular body 2a constituting this housing 2; puncturing opening 2c provided at the tip of this tubular body 2a; laser puncturing unit 3 provided in housing 2; electrical circuit section 5 connected to this laser puncturing unit 3; and battery 6 that supplies power to this electrical circuit section 5 and laser puncturing unit 3.

Now, operation of the above-mentioned laser puncturing apparatus 1 will be described.

FIG.2 is a perspective view explaining a usage sate of laser puncturing apparatus 1.

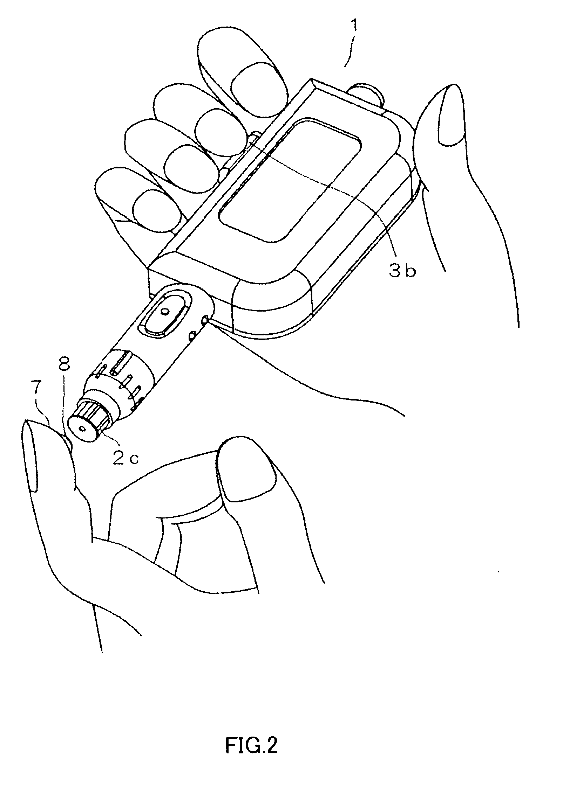

As shown in FIG.2, the user holds laser puncturing apparatus 1 by the right hand and touches laser puncturing apparatus 1 to skin 7 of the left hand. Then, the user presses puncturing button 3b. Then, laser light 3a is emitted from laser puncturing unit 3 (see FIG.1). This laser light 3a punctures skin 7. Blood 8 exudes from skin 7 by this puncturing. Then, the blood sugar level of this exuded blood 8 is measured by a blood test apparatus (not shown).

This puncturing unit 3 requires a large amount of power every time puncturing is performed. Therefore, if battery 6 is consumed and the remaining battery level of battery 6 gets low, the patient judges that fact and has to replace battery 6 in each case accordingly.

    Patent Document 1: Published Japanese Translation of PCT application No.2004-533866
    Patent Document 2: Korean registered utility model No. 20-0420950 (Y1)

Disclosure of Invention

Problems to be Solved by the Invention

However, in such a conventional laser puncturing apparatus, since a large amount of power is consumed by one puncturing operation, it is necessary to replace the battery periodically, or in case of a secondary battery, it is necessary to charge the battery periodically. Therefore, unless spare batteries and the battery charger are prepared to use at any time, the patient might not perform puncturing when the remaining battery level is low. Particularly, when going out, the patient has to carry batteries and the battery charger, so that baggage increases and the burden is bigger. If it is not possible to perform puncturing, blood can not be sampled, so that it is difficult for the patient to administer the appropriate dose of insulin. Therefore, degradation of the condition can be expected.

The present invention has solved the above-described problem. It is therefore an object of the invention is to provide a puncturing apparatus that displays the number of remaining puncturing operations or the number of remaining blood tests, a blood test apparatus and a puncturing method.

Means for Solving the Problem

The puncturing apparatus according to the present invention has a configuration including: a housing; a puncturing section that is provided in the housing and that punctures skin; a battery that supplies power to the puncturing section; a remaining battery level measuring section that measures a remaining level of the battery; a power consumption measuring section that measures electric power consumed by the battery for puncturing operation of the puncturing section; a remaining puncturing calculation section that calculates the number of remaining puncturing operations that can be performed by the puncturing section, based on the remaining battery level and the power consumption that have been measured; and an informing section that informs the number of remaining puncturing operations calculated by the remaining puncturing calculation section.

The blood test apparatus according to the present invention has a configuration including: a puncturing section that punctures skin with laser light; a blood test section that tests blood; a battery that supplies power to the puncturing section and the blood test section; a remaining battery level measuring section that measures a remaining battery level; a power consumption measuring section that measures electric power consumed by the battery for puncturing operation of the puncturing section; a remaining blood test calculation section that calculates the number of remaining blood tests that can be performed by the blood test section, based on the remaining battery level and the power consumption that have been measured; and an informing section that informs the number of remaining blood tests calculated by the remaining blood test calculation section.

The puncturing method according to the present invention includes: measuring a remaining battery level that supplies power to a puncturing section; measuring electric power consumed by the battery for puncturing operation of the puncturing section; calculating the number of remaining puncturing operations that can be performed by the puncturing section, based on the remaining battery level and the power consumption that have been measured; and informing the number of remaining puncturing operations that has been calculated.

The puncturing method according to the present invention includes: measuring a remaining battery level that supplies power to a negative pressure section and a puncturing section; measuring electric power consumed by the battery for negative pressure creating operation of the negative pressure section and for puncturing operation of the puncturing section; calculating the number of remaining puncturing operations that can be performed by the puncturing section, based on the remaining battery level and the power consumption that have been measured; and informing the number of remaining puncturing operations that has been calculated.

The puncturing method according to the present invention includes: measuring a remaining battery level that supplies power to a blood test section that measures components of blood, a negative pressure section that creates a negative pressure and a puncturing section; measuring electric power consumed by the battery for negative pressure creating operation of the negative pressure section, for puncturing operation of the puncturing section and for operation of the blood test section; calculating the number of remaining blood tests that can be performed by the blood test section, based on the remaining battery level and the power consumption that have been measured informing the number of remaining blood tests that has been calculated.

Advantageous Effects of Invention

According to the present invention, the number of remaining puncturing operations that can be performed by the puncturing means is calculated, based on the remaining battery level and the power consumption that are measured, so that the display section can display the number of remaining puncturing operations that can be performed or the number of remaining blood tests. By this means, when going out, the patient charges the battery or replaces the battery in advance when the number of remaining puncturing operations is less than the number of times of use while going out, and consequently such a problem that puncturing cannot be performed unexpectedly in a place where the patient has gone is prevented. Therefore, it is possible to administer the appropriate dose of insulin, so that degradation of the condition can be prevented.

In addition, the battery can be run down at the finish in ca se of a primary battery, so that economical efficiency can be provided.

Brief Description of Drawings

FIG.1 is a cross sectional view of the configuration of a conventional laser puncturing apparatus;
FIG.2 is a perspective view explaining a usage state of the conventional laser puncturing apparatus;

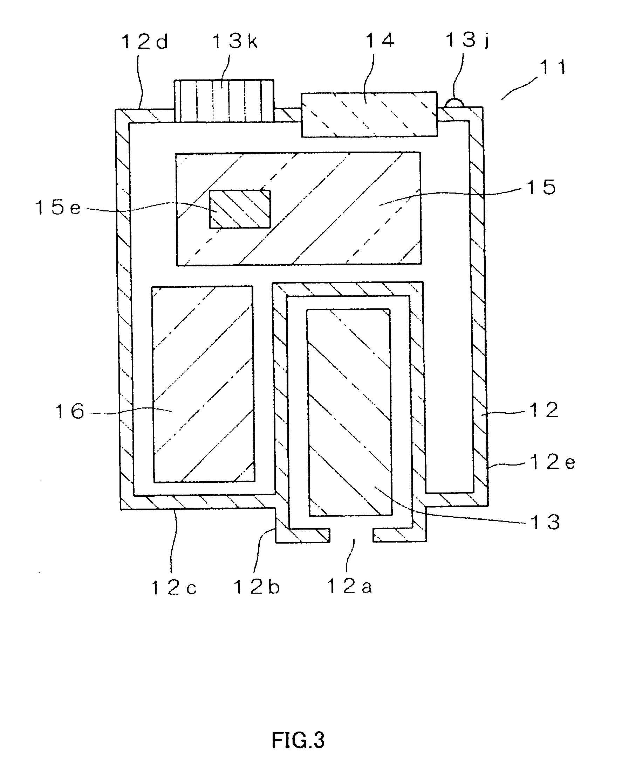

FIG.3 is a cross sectional view showing the configuration of a puncturing apparatus according to embodiment 1 of the present invention;

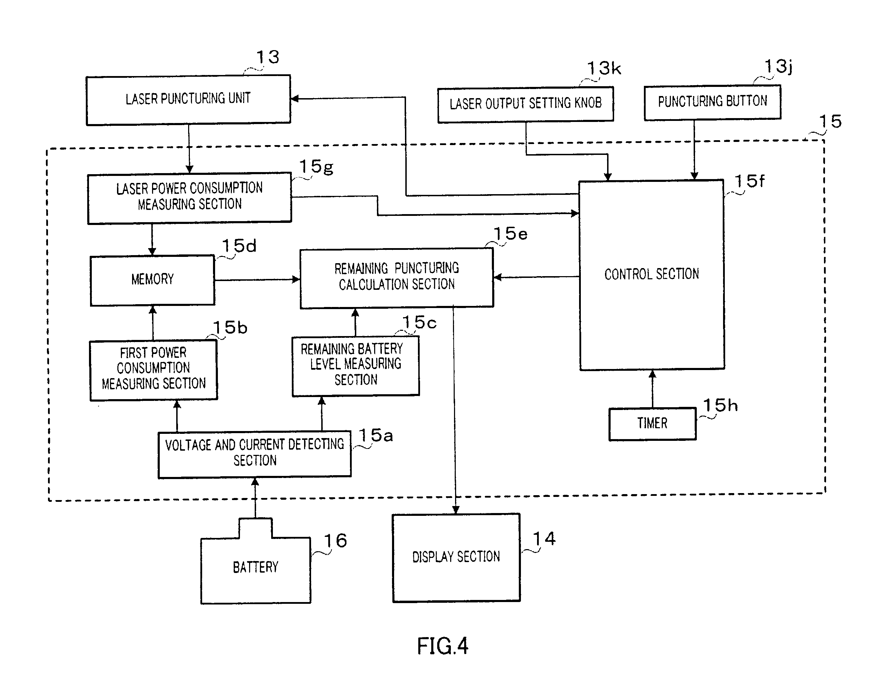

FIG.4 is a block diagram of an electrical circuit section and its neighborhood, in the puncturing apparatus;

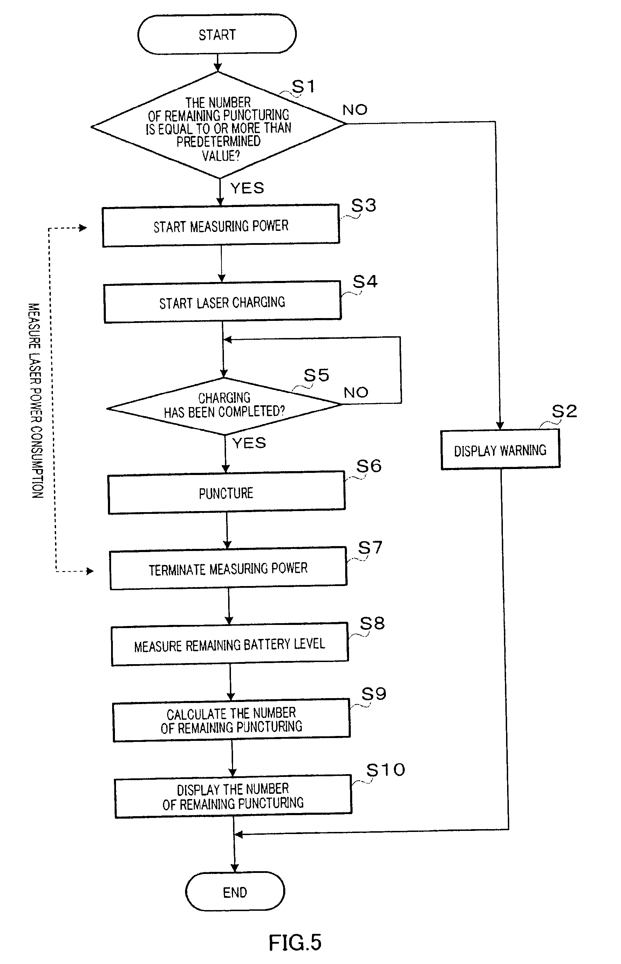

FIG.5 is a flow chart showing operation steps of the puncturing apparatus;

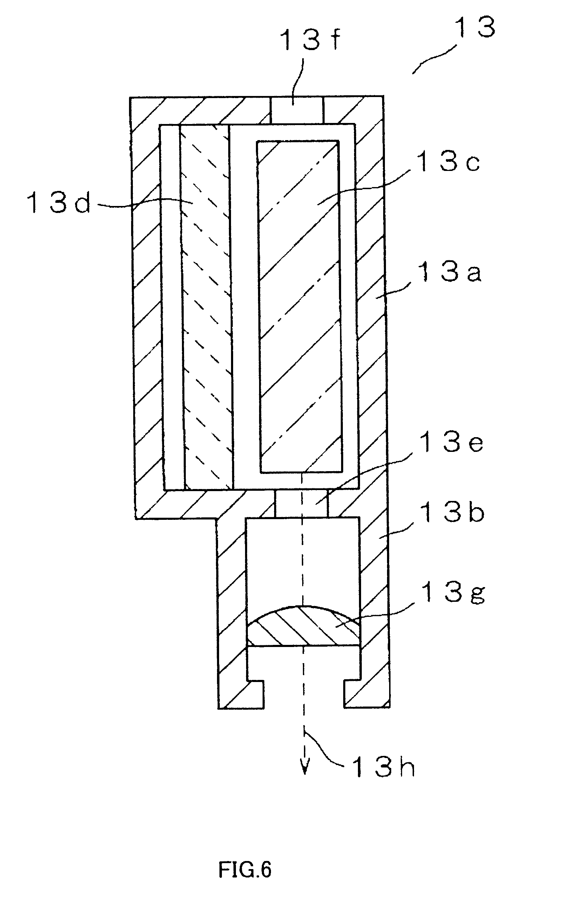

FIG.6 is a cross sectional view of a laser puncturing unit as an example of the puncturing apparatus;

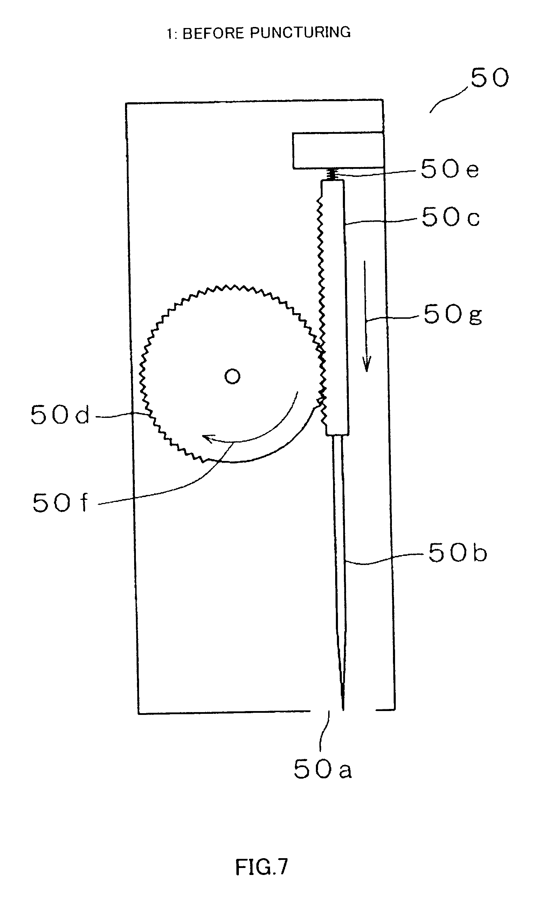

FIG.7 is a cross sectional view of a first needle puncturing unit of a puncturing apparatus according to embodiment 2;

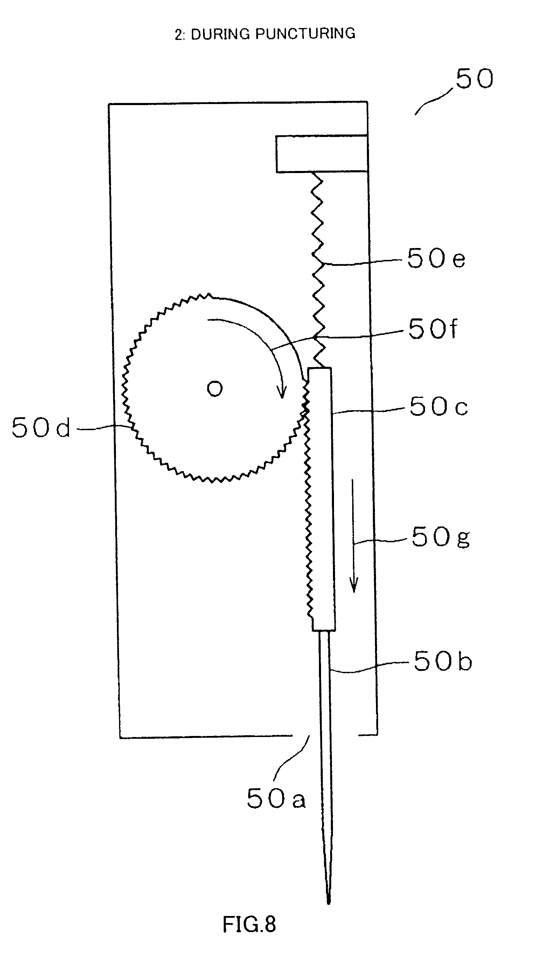

FIG.8 is a cross sectional view of the first needle puncturing unit when puncturing, of the puncturing apparatus ;

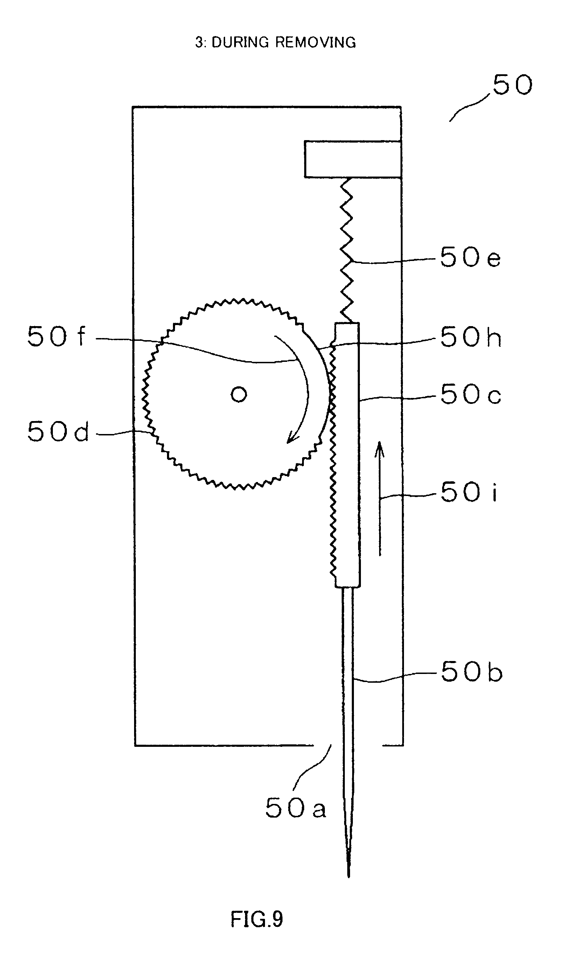

FIG.9 is a cross sectional view of the first needle puncturing unit when being removed, of the puncturing apparatus ;

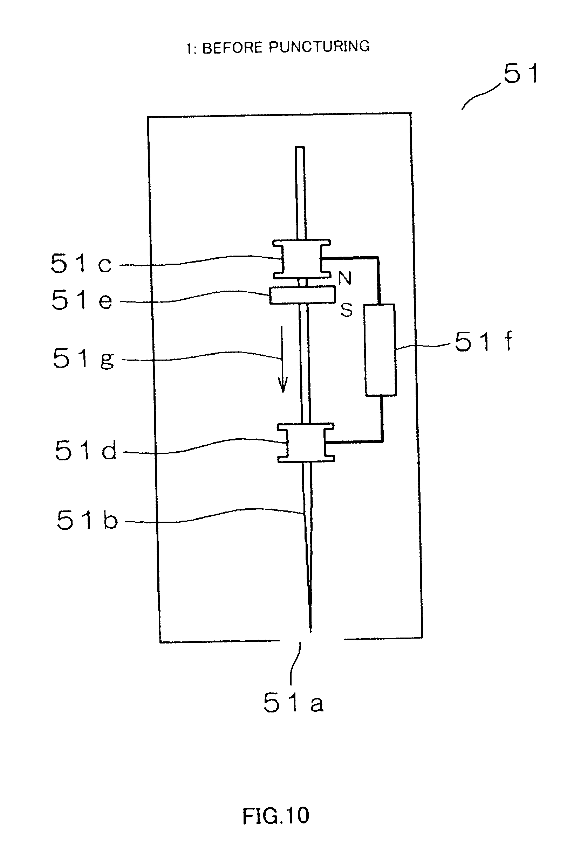

FIG.10 is a cross sectional view of a first needle puncturing unit before puncturing, of a puncturing apparatus according to embodiment 3 of the present invention;

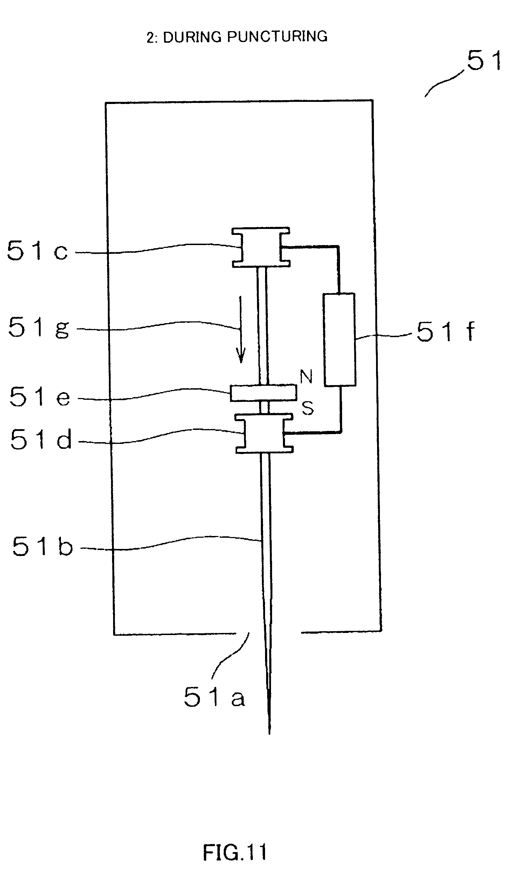

FIG.11 is a cross sectional view of the first needle puncturing unit when puncturing, of the puncturing apparatus ;

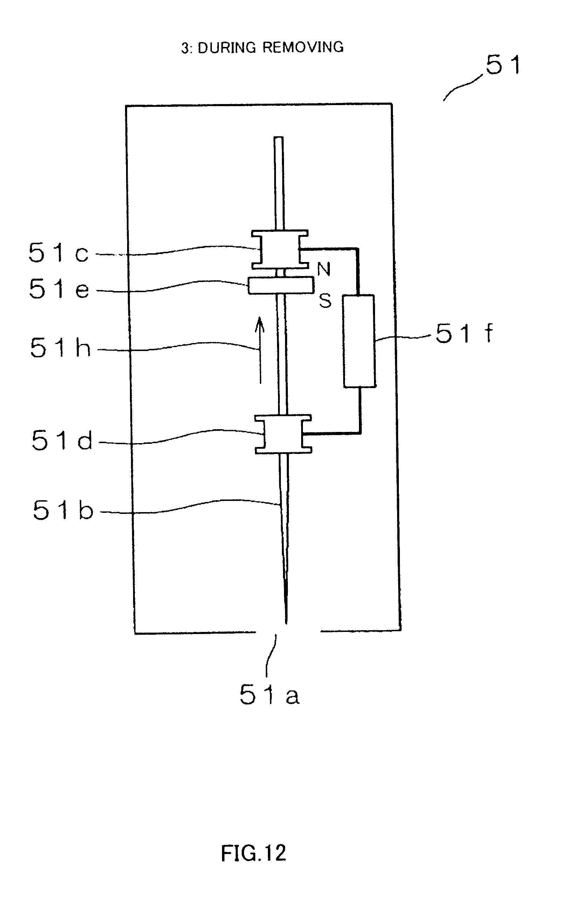

FIG.12 is a cross sectional view of the first needle puncturing unit when being removed, of the puncturing apparatus ;

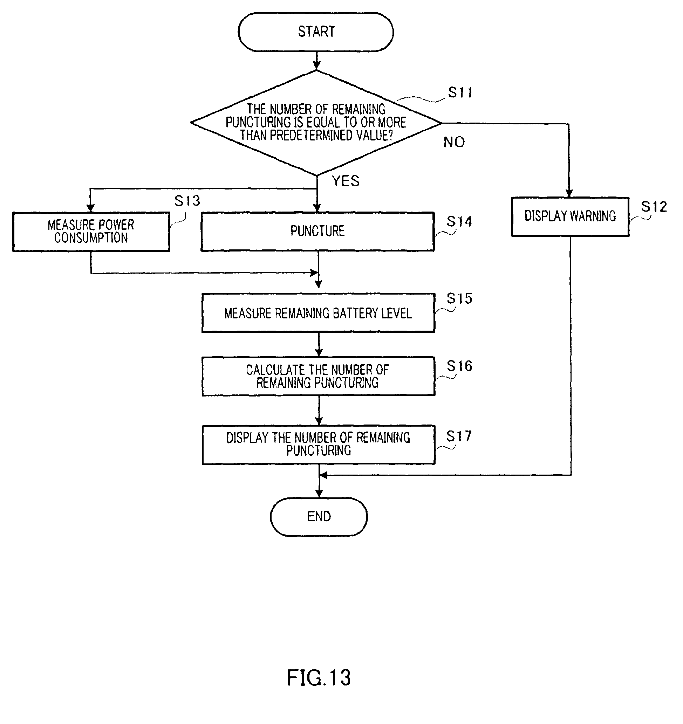

FIG.13 is a flow chart showing operation of the puncturing apparatus in a case where the first needle puncturing unit is used as the puncturing means according to embodiment 2, or in a case where the second needle puncturing unit is used as the puncturing means according to embodiment 3;

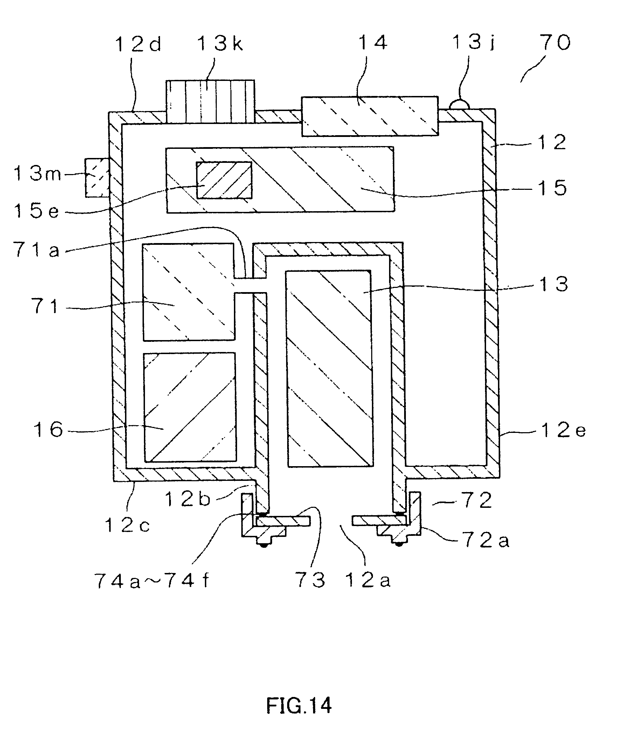

FIG.14 is a cross sectional view showing the configuration of a puncturing apparatus having a blood testing section according to embodiment 4 of the present invention;

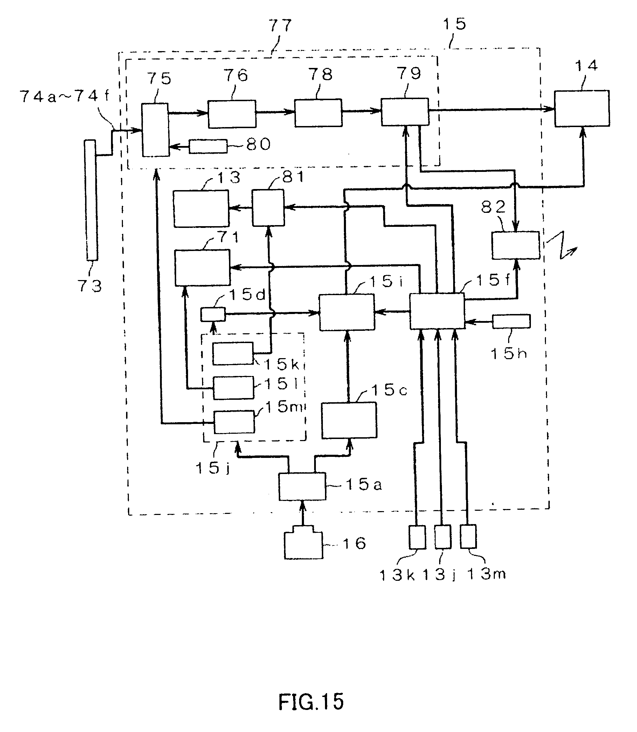

FIG.15 is a block diagram of an electrical circuit section built in the puncturing apparatus having the blood test section and its neighborhood;

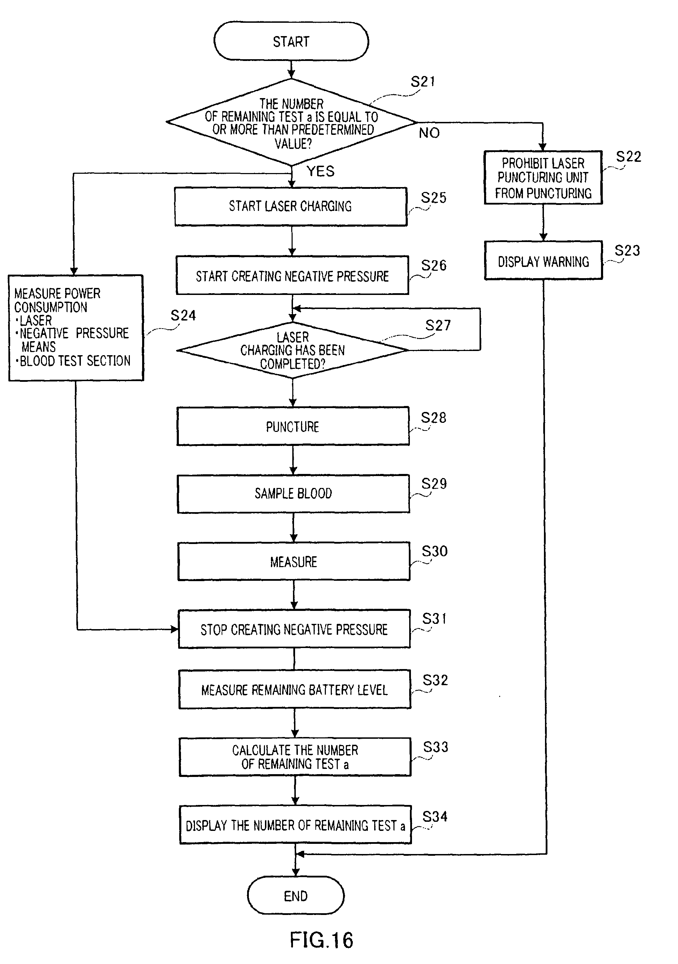

FIG.16 is a flowchart showing operation of the puncturing apparatus having the blood test section;

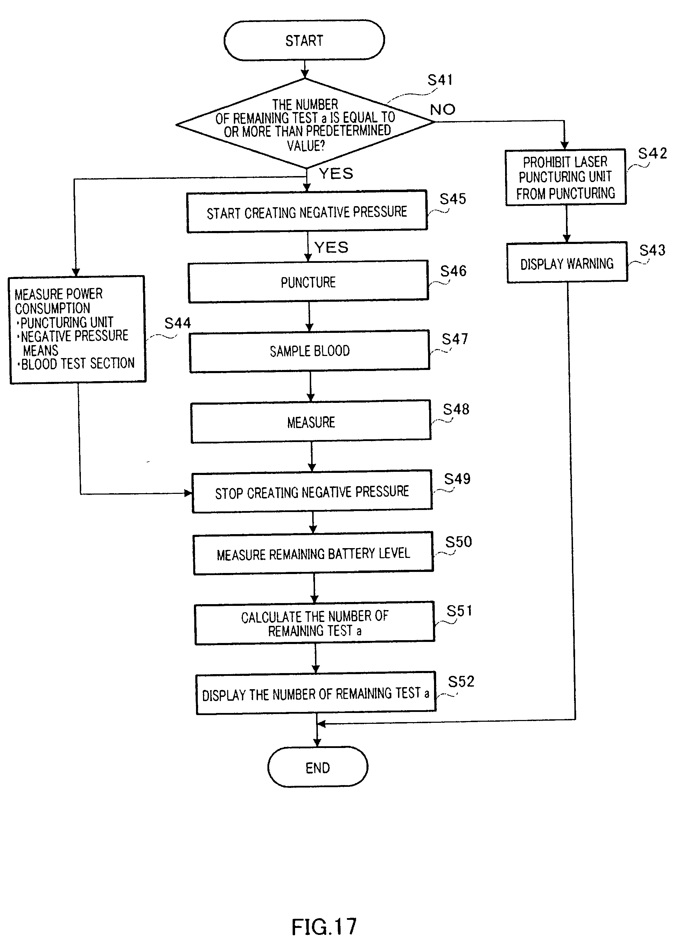

FIG.17 is a flowchart showing operation of the puncturing apparatus having the blood test section;

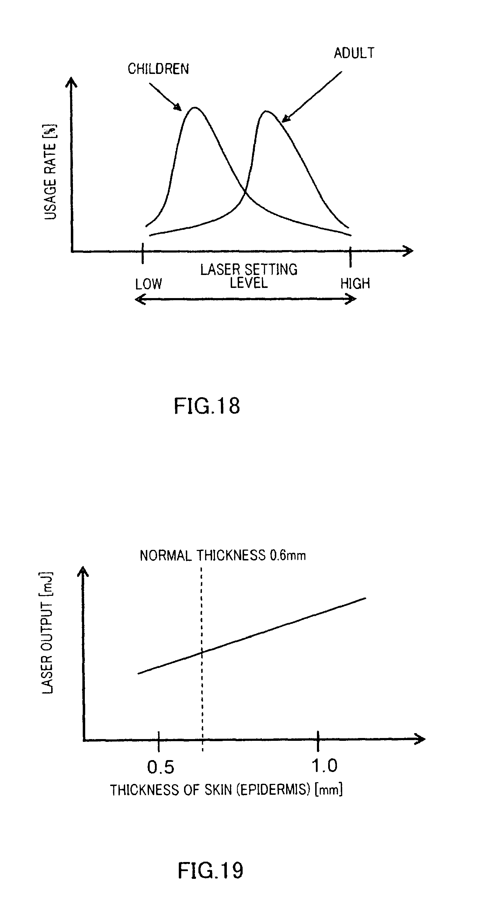

FIG. 18 is a drawing showing the laser setting level measured by the inventers and its usage rate (%);

FIG.19 is a drawing showing the relationship between the thickness of skin (mm) and the laser output (average) (mJ) required for puncturing;

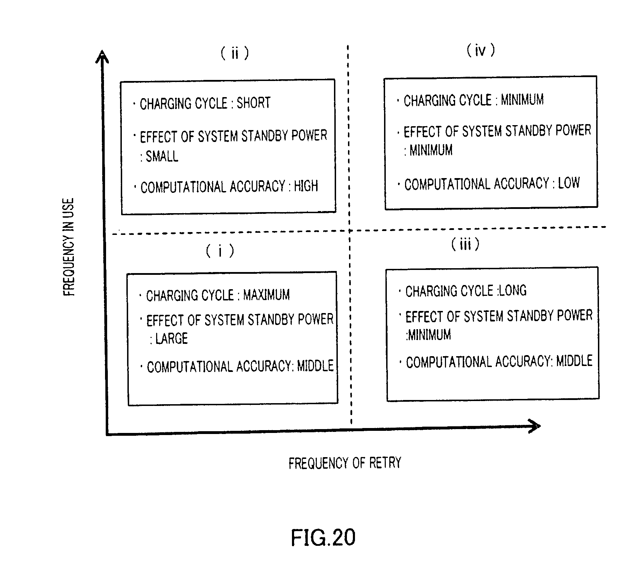

FIG.20 is a drawing explaining the tendency of the charging cycle of batteries and the computational accuracy of the number of remainders, based on the relationship between the frequency in use and the number of retries;

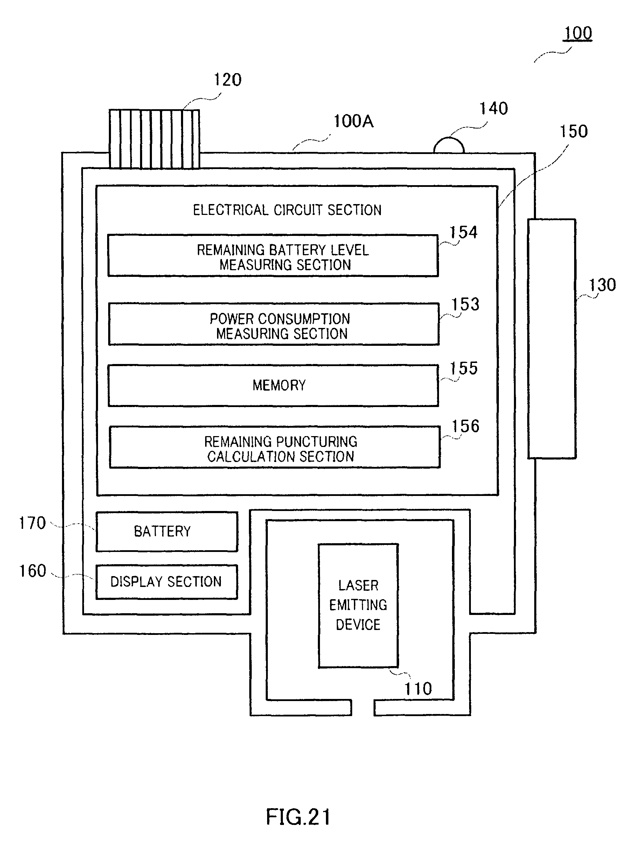

FIG.21 is a schematic diagram showing the entire configuration of a laser puncturing apparatus according to embodiment 5 of the present invention;

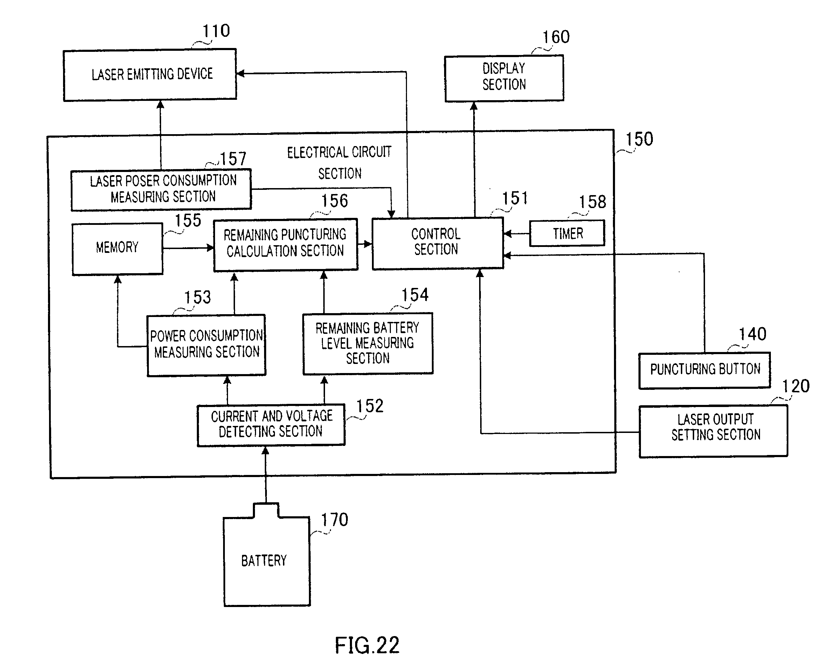

FIG.22 is a block diagram of an electrical circuit section and its periphery in the laser puncturing apparatus;

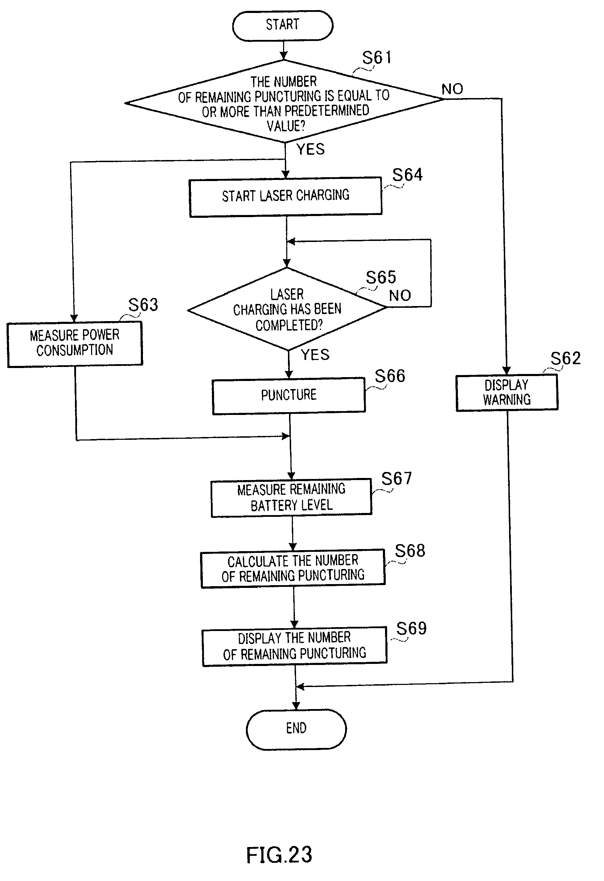

FIG.23 is a flowchart showing display operation of the number of remaining puncturing operations of the laser puncturing apparatus according to embodiment 5;

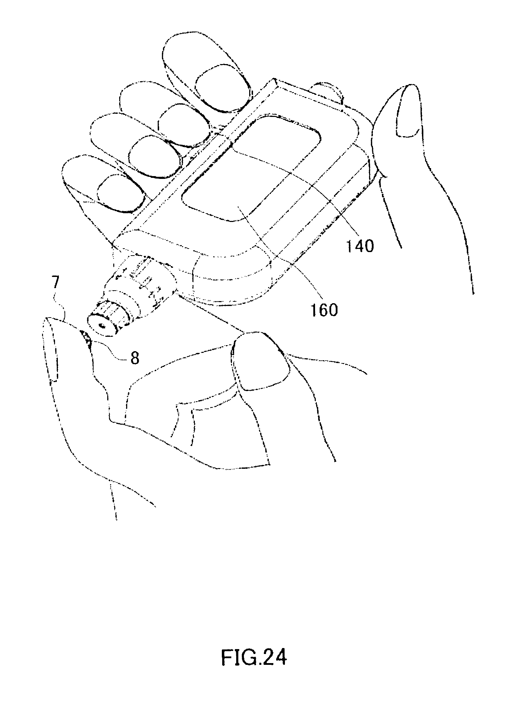

FIG.24 is a perspective view explaining a usage state of the laser puncturing apparatus;

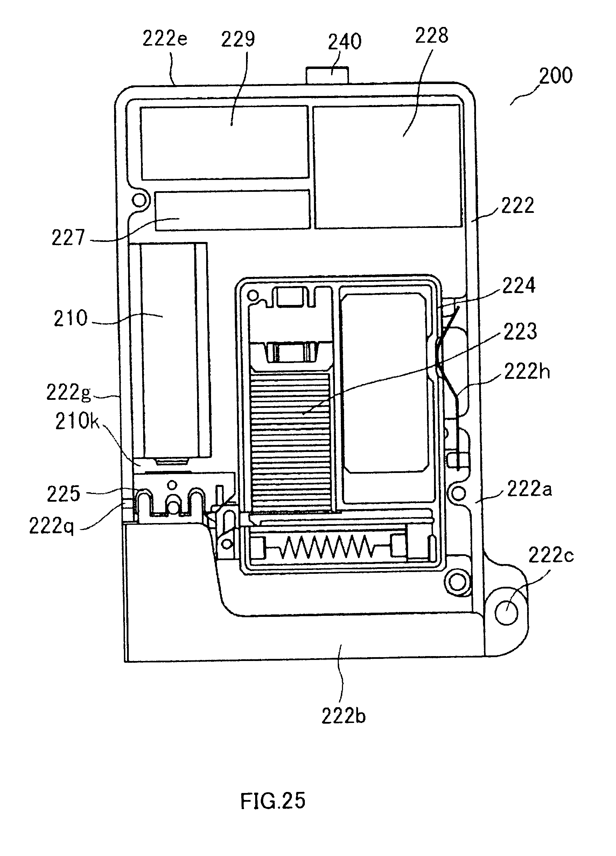

FIG.25 is a cross sectional view of a blood test apparatus according to embodiment 6 of the present invention;

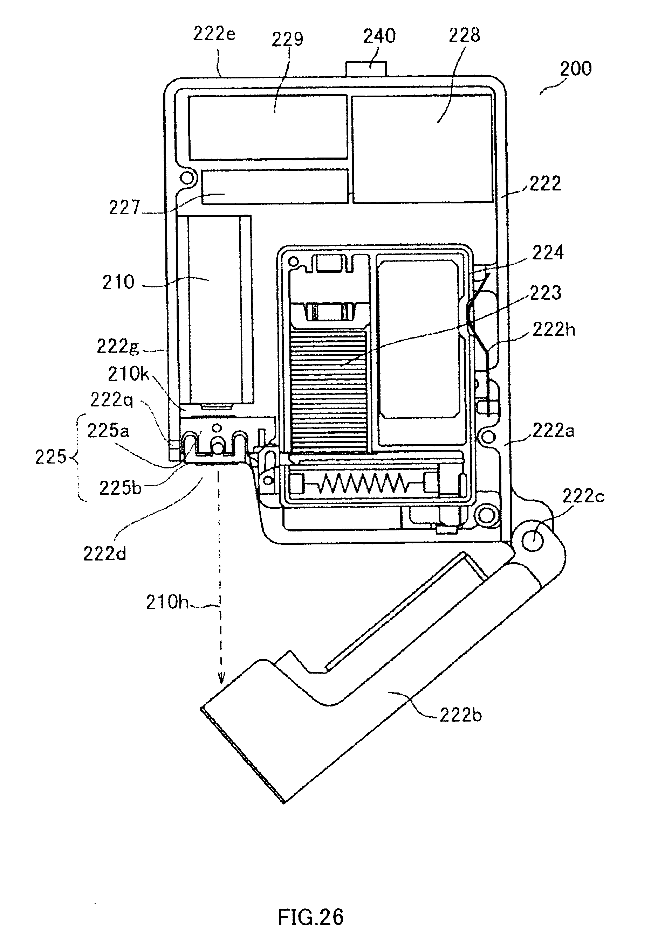

FIG.26 is a cross sectional view of the blood test apparatus ;

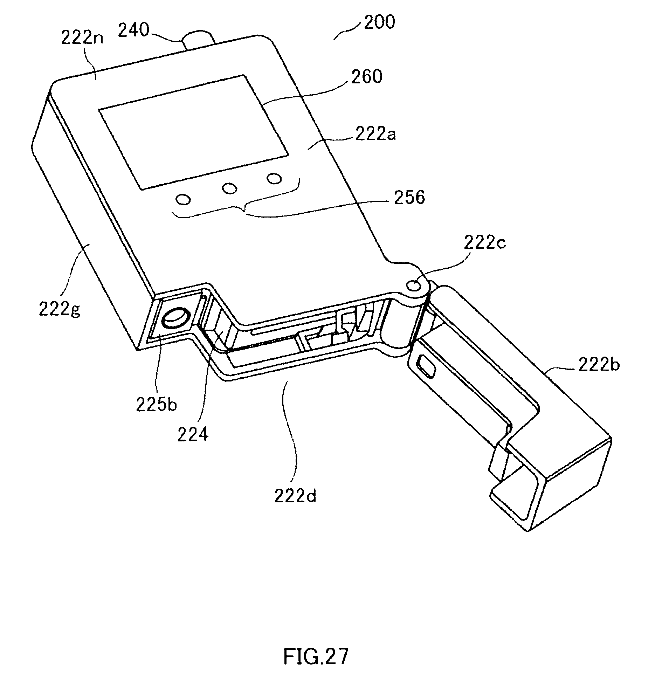

FIG.27 is a perspective view of the blood test apparatus ;

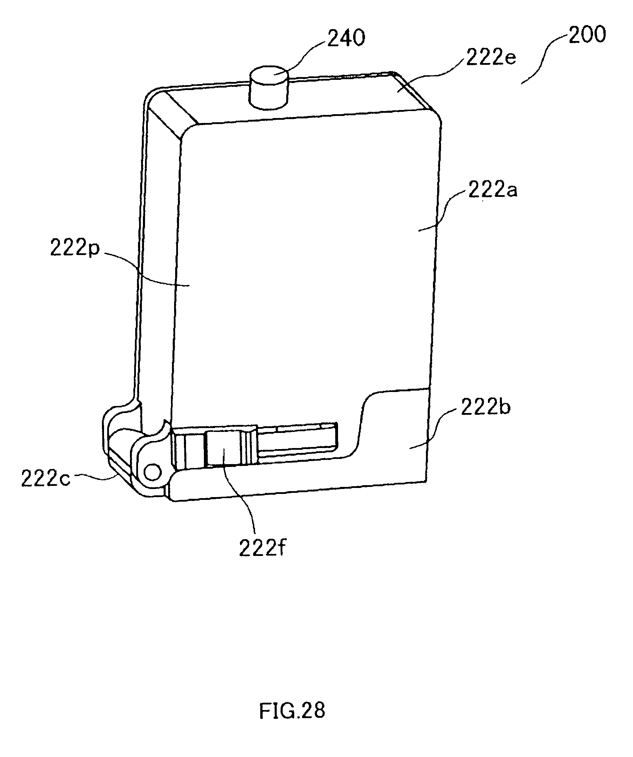

FIG.28 is a perspective view of the blood test apparatus from the back surface;

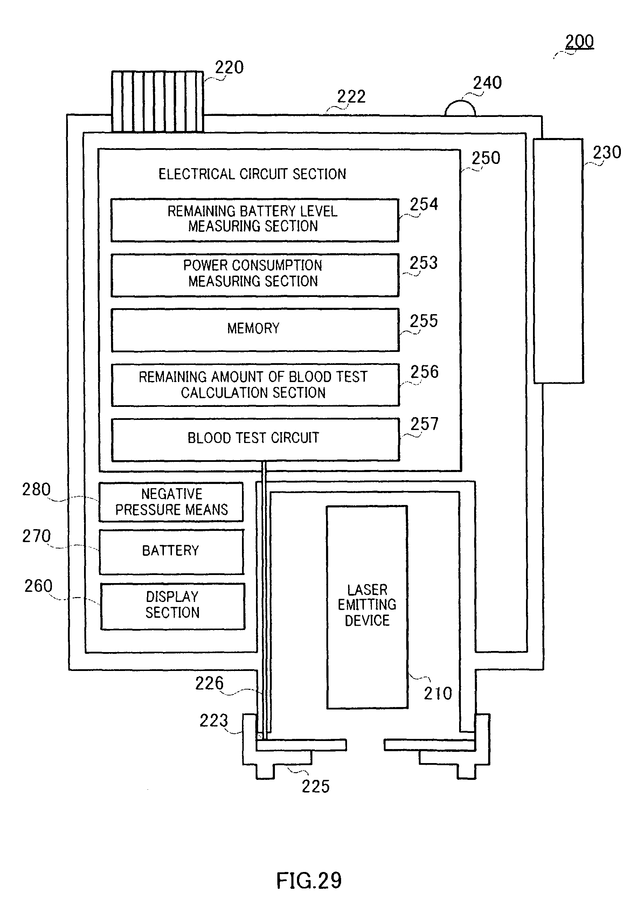

FIG.29 is a schematic diagram showing the entire configuration of the blood test apparatus according to embodiment 6;

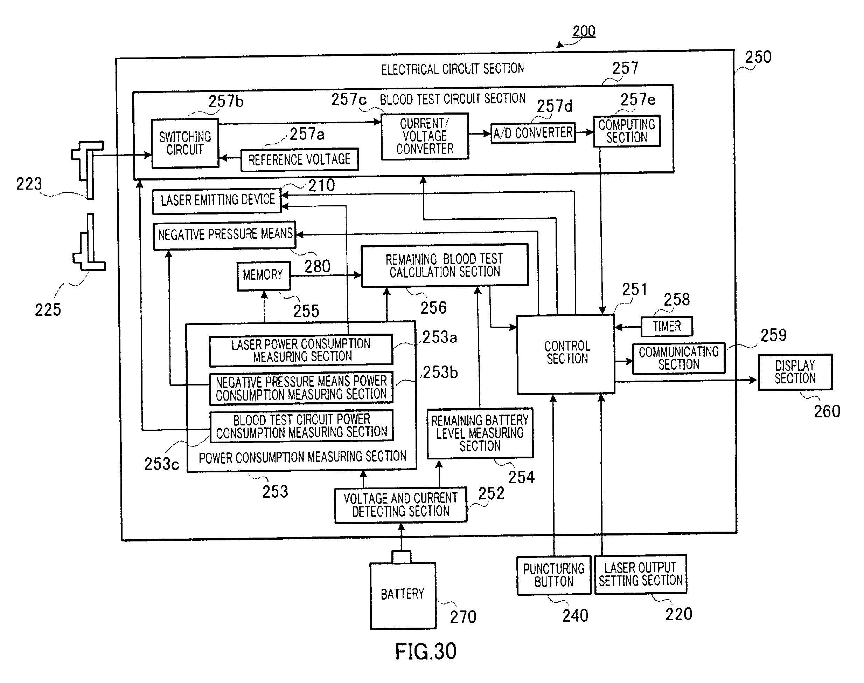

FIG.30 is a block diagram of an electrical circuit section and its periphery in the blood test apparatus according to embodiment 6; and

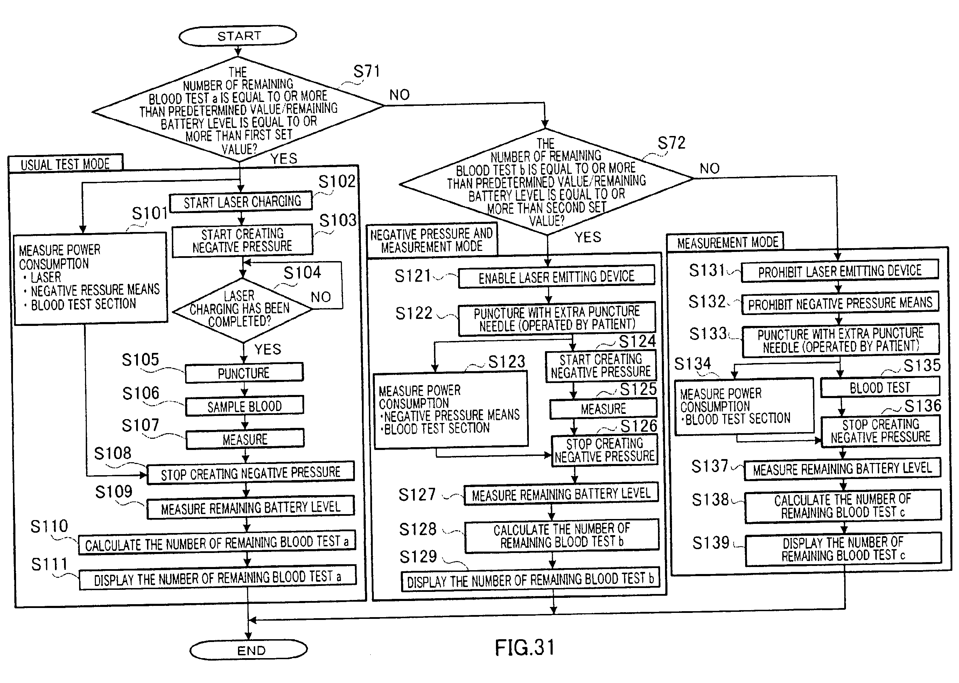

FIG.31 is a flowchart showing display operation of the number of times of blood tests of the blood test apparatus according to embodiment 6.

Best Mode for Carrying Out the Invention

Now, the embodiments of the present invention will be described in detail with reference to the drawings. Here, in description of each embodiment, directions such as the vertical direction is defined based on the blood test apparatus in use.

(Embodiment 1)

Fig. 3 is a cross sectional view showing the configuration of a puncturing apparatus according to embodiment 1 of the present invention. The present embodiment is applied to a laser puncturing apparatus as a puncturing means.

In FIG.3, puncturing apparatus 11 has housing 12 made of resin, and one side 12c of housing 12 constitutes a cylindrical shaped-tubular body 12b having puncturing opening 12a. Laser puncturing unit 13, which is a puncturing means, is built in tubular body 12b. In addition, display section 14, puncturing button 13j and laser output setting knob 13k are provided on the other side 12d of housing 12.

Display 14 is utilized to display the conditions of puncturing apparatus 11 and messages. In addition, puncturing button 13j is a button to start puncturing operation of puncturing apparatus 11, and this puncturing button 13j is pressed down, so that laser light is emitted from laser puncturing unit 13.

Laser output setting knob 13k is a knob to adjust and set output of laser puncturing unit 13, and this laser output setting knob 13k is adjusted, so that the optimal puncturing output (power) can be provided to the patient.

Laser puncturing unit 13 is composed of an excitation light source, a laser rod, a partially reflecting mirror and a total reflecting mirror that are arranged on both sides of the laser rod, focusing lens and so forth. This laser puncturing unit 13 will be described in detail later.

Electrical circuit section 15 that controls the operation of laser puncturing unit 13 is housed between laser puncturing unit 13 and the other side 12d of housing 12. In addition, buttery 16 is housed replaceably next to tubular body 12b. A primary battery and a secondary battery may be used as battery 16. A lithium battery, an oxyride battery, an alkaline battery and so forth are used as a primary battery, meanwhile, a nickel-cadmium battery, a nickel hydride battery, a lithium-ion battery and so forth are used as a secondary battery. In the present embodiment, the lithium-ion battery, which is a secondary battery is employed.

FIG.4 is a block diagram of an electrical circuit section 15 and its neighborhood, in the puncturing apparatus according to embodiment 1.

In FIG.4, electrical circuit section 15 has a configuration including: control section 15f configured by a microcomputer; memory 15d; timer 15h; laser power consumption measuring section 15g that measures the power consumption of laser puncturing unit 13; voltage and current detecting section 15a that detects the voltage and the current of buttery 16; first power consumption measuring section 15b that measures the power consumption of the entire system; remaining battery level measuring section 15c that measures the remaining battery level; and remaining puncturing calculation section 15e. Operation information from laser output setting knob 13k and puncturing button 13j is inputted to control section 15f of electrical circuit section 15. In addition, remaining puncturing calculation section 15e outputs, to display section 14 made of LCD, the number of remaining puncturing operations that has been calculated.

Voltage and current detecting section 15a detects a voltage of battery 16 and a current flowing out from battery 16 and outputs the voltage and current detection result to first power consumption measuring section 15b and remaining battery level measuring section 15c.

First power consumption measuring section 15b calculates the power consumption by multiplying current value (A) flowing out from buttery 16 for one puncturing operation, voltage (V) of buttery 16 and time (h) required to perform puncturing.

Memory 15d stores and accumulates data of power consumption measured in power consumption measuring section 15b every time puncturing is performed.

Data of the remaining battery level calculated in remaining battery level measuring section 15c and data of the number of remaining puncturing operations calculated in remaining puncturing calculation section 15e are also stored in memory 15d.

Remaining buttery level measuring section 15c is configured by an integrated circuit dedicated to measurement of the remaining battery level and multiplies the voltage value and the current value in the unloaded condition. Then, remaining battery level measuring section 15c calculates the remaining battery level by subtracting this multiplied value from the capacity of battery 16 at the time battery 16 is replaced. Here, also data of the remaining battery level measured by remaining battery level measuring section 15c may be stored in memory 15d every time puncturing is performed.

Remaining puncturing calculation section 15e calculates the number of remaining puncturing operations, based on the output of remaining battery level measuring section 15c and the output of memory 15d.

Specifically, remaining puncturing calculation section 15e calculates the number of remaining puncturing operations by dividing the remaining battery level measured in remaining battery level measuring section 15c by the power consumption for one puncturing operation of the puncturing means (laser puncturing unit, etc.), which is measured in first power consumption measuring section 15b. In this case, as the power consumption for one puncturing operation, the average of the power consumption of every puncturing stored in memory 15d. The power consumption for one puncturing operation differs depending on the usage condition and state of each patient. By averaging the power consumption of every puncturing of each patient, it is possible to calculate the number of remaining puncturing operations customized to each patient, and it is possible to calculate more accurately the number of remaining puncturing operations more suitable for each patient.

Display section 14 displays the number of remaining puncturing operations that has been calculated by remaining puncturing calculation section 15e, based on a command from control section 15f.

The patient can know accurately the number of remaining puncturing operations that is optimized for the patient and that is reliable. By this means, for example, the patient checks the number of remaining puncturing operations before going out and charges the battery if the number of remaining puncturing operations is less than the number of times of use while going out, and consequently such a trouble that puncturing cannot perform in a place where the patient has gone, can be prevented.

The above-described number of remaining puncturing operations may be calculated by dividing the remaining battery level by the fixed value or the set value of the power consumption corresponding to laser output setting knob 13k connected control section 15f. In this case, display section 14 and electrical circuit section 15 also consume electric power, so that the power consumption slightly fluctuates by just that. However, since the power consumption of laser puncturing unit 13 is overwhelmingly larger than the power consumption of display section 14 and electrical circuit section 15 (about hundredfold of the power consumption of electrical circuit section 15), the remaining battery level can be calculated using the fixed value or the set value in consideration of only the power consumption of laser puncturing unit 13.

In this instance, a case where the power consumption can be expected based on the rated value of the puncturing apparatus in advance and a case where puncturing is regularly performed are assumed, so that the number of remaining puncturing operations can be calculated using the fixed value and the set value, instead of the average of data accumulated in memory 15d as described above.

Puncturing button 13j is connected to control section 15f, and when this puncturing button 13j is pressed down, laser puncturing unit 13 is activated to emit laser light 13h (see FIG.6).

Laser power consumption measuring section 15g measures the power consumed by laser puncturing unit 13 every time puncturing is performed. This measurement result is supplied to remaining puncturing calculation section 15e through control section 15f, and can be used for further accurate calculation of the number of remaining puncturing operations by remaining puncturing calculation section 15e. In addition, the measurement result may be stored in memory 15d.

Laser output setting knob 13k is a knob to adjust, in a multiple-step form, the level of laser output to customize to the patient. In the present embodiment, the output level of laser output setting knob 13k can be set to approximately even 8 steps of laser output from 100 mJ to 600 mJ. By this means, the output level can be lower for patients having thin skin, such as children and women, meanwhile, the output level can be higher for patients having thick skin, such as men, so that it is possible to perform puncturing customized to various patients. In addition, although a knob is employed in the present embodiment, a dial may be employed, and when a touch panel is used to select from the display screen of display section 14 or when a touch panel is mounted to the screen of display section 14, input may be performed through the touch panel.

FIG.5 is a flow chart showing operation steps of puncturing apparatus 11 using laser puncturing unit 13. In the figure, S shows each step of the flow.

In step S1, first, data of the number of remaining puncturing operations stored in memory 15d is checked. At this time, whether the number of remaining puncturing operations is equal to or more than a predetermined value (a predetermined number of times) is determined.

Here, remaining battery level measuring section 15c measures the remaining battery level 16 and further recalculates data of the number of remaining puncturing operations based on data of the previous power consumption stored in memory 15d, and if the recalculation result is different from the data of the number of remaining puncturing operations stored in memory 15d, the control section determines that some error occurs and display section 14 may give warning by a warning indication and an alarm (not shown). By this mean, the reliability of number of remaining puncturing operations can be further improved. Here, when the apparatus is used for the first time and there is no data of number of remaining puncturing operations and data of power consumption in memory 15d, the number of remaining puncturing operations can be calculated from data of initial power consumption stored in the memory in advance and the remaining battery level 16 calculated by remaining battery level measuring section 15.

If the number of remaining puncturing operations is less than the predetermined number of times, control section 15f makes display section 14 give warning by a warning indication and an alarm (not shown) in step S2. As for the warning indication in step S2, for example, display 14 displays the indication to replace battery 16 with battery 16a and the instruction to charge battery 16 when battery 16 is a secondary battery. Here, the warning indication is to give warning to the patient and is not limited to visual indication. Audio indication such as an electronic buzzer and audio response, and touch sense indication such as vibration may be applicable. After waning indication is displayed, this flow ends. In the present embodiment, when the number of remaining puncturing operations is less than one, puncturing by the puncturing means (laser puncturing unit 13 here) in that state is forcibly stopped (prohibited).

When the number of remaining puncturing operations is equal to or more than the predetermined number of times in step S1 described above, first power consumption measuring section 15b starts measuring power consumption.

In step S4, control section 15f starts charging laser puncturing unit 13 to emit laser light.

In step S5, control section 15f determines whether laser puncturing unit 13 has been charged.

In step S5, when charging of laser puncturing unit 13 is completed, control section 15f makes display section 14 display to the patient that it is possible to perform puncturing in step S6.

When the patient presses puncturing button 13j according to this display, the step moves to step S6.

In step S6, control section 15f outputs a command to puncture to laser puncturing unit 13 and laser puncturing unit 13 emits laser light 13h. Laser light 13h emitted from laser puncturing unit 13 is focused by focusing lens 13b and is irradiated to skin 7 (not shown, hereinafter the same meaning shall apply but described laser in embodiment 4) of the patient. By this means, the skin is punctured by ablation and blood 8 exudes from skin 7.

In step S7, first power consumption measuring section 15b stops measuring the power consumption.

In step S8, remaining battery level measuring section 15c measures the remaining battery level 16, and the measurement value of the remaining battery level is outputted to remaining puncturing calculation section 15e. Here, the remaining battery level can be more accurately measured by measuring the voltage drop in the loaded condition for the battery, in order to measure the remaining battery level, so that this voltage drop information may be usable. For that purpose, voltage drop information when the load on the battery during charging is larger may be stored in the memory.

In step S9, remaining puncturing calculation section 15e calculates the number of remaining puncturing. Data of first power consumption measurement measured by first power consumption measuring section 15b and data of the number of remaining puncturing operations are stored in memory 15d.

In step S10, control section 15f makes display section 14 display the number of remaining puncturing operations that has been calculated by remaining puncturing calculation section 15e and ends this flow.

In addition, here, when the number of remaining puncturing operations is equal to or less than a predetermined value, display section 14 may give warning by a warning indication and an alarm to indicate that the number of remaining puncturing operations is small and by a response massage, in order to prompt the patient to charge and replace the secondary battery.

FIG.6 is a cross sectional view of laser puncturing unit 13, which is an example of puncturing means.

In FIG.6, laser puncturing unit 13 is composed of oscillating tube 13a and cylindrical tubular body 13b coupled to this oscillating tube 13a, where cylindrical tubular body 13b is located in front of oscillating tube 13a Er:YAG (yttrium, aluminum, garnet) laser crystal 13c and flash light source 13d (used as an example of light source) are housed in oscillating tube 13a. Partially transmitting mirror 13e having a transmissivity of 1% to 10% is mounted at one end of oscillating tube 13a, and total reflecting mirror 13f is mounted at the other end of oscillating tube 13a. Focusing lens 13g is mounted in tubular body 13b in front of partially transmitting mirror 13e, and is arranged such that laser light 13h focuses on a predetermined puncturing position near the surface of skin of the user.

Next, operation of laser puncturing unit 13 will be described.

In order to generate laser light, first, it is necessary to apply a high voltage to flash light source 13d, which is an excitation light source. Therefore, laser puncturing unit 13 is provided with a high voltage capacitor (not shown), and this high voltage capacitor is charged (see FIG.5). When the charging voltage for this capacitor reaches a predetermined voltage, charging is completed, so that a predetermined voltage is applied to both ends of flash light source 13d connected to the capacitor.

In the state where the capacitor has been charged, puncturing button 13j (see FIG.3 and FIG.4) is pressed. By this means, a trigger signal is outputted from control section 15f to flash light source 13d to emit light. This light-emitting allows Er:YAG laser crystal 13s to excite and a resonator configured by total reflecting mirror 13f and partial transmitting mirror 13e amplifies laser energy. Part of this amplified energy passes through partial transmitting mirror 13e by induced emission. This is laser light 13h. This laser light 13h passing though partial transmitting mirror 13e is condensed by lens 13g and focuses on the position in skin 7 (see FIG.14, etc.) Here, the appropriate focal depth (i.e. focal position) for puncturing is 0.1 mm to 1.5 mm from the surface of skin 7. In the present embodiment, the puncturing depth is 0.5 mm. Blood 8 exudes from punctured skin 7.

As described above, the present embodiment employs laser puncturing unit 13. Therefore, in general use, it is not necessary to replace a puncturing needle, differently from a puncturing apparatus using a puncturing needle, so that preparations before puncturing can be simplified. In addition, skin 7 is not contact with puncturing unit 13, so that there is no risk of infection and it is possible to perform puncturing in safety. Moreover, since the puncturing depth can be adjusted by adjusting the charging voltage, so that puncturing customized to each patient, and consequently the patient feels little pain.

In addition , although a negative pressure means has not been described yet in the present embodiment, the negative pressure means can be employed as with another embodiment described later. The negative pressure means will be described in detail in another embodiment described later.

(Embodiment 2)

Embodiment 2 employs a first needle puncturing unit instead of laser puncturing unit 13 (see FIG.6) built in puncturing apparatus 11 of FIG.3. In this first needle puncturing unit, the puncturing needle is not driven by the force of a spring in the conventional way, but is driven by a motor to perform puncturing. By this means, the movement speed of the puncturing needle, that is, the puncturing speed can be controlled, so that it is possible to perform puncturing with further little pain.

FIG.7 to FIG.9 are cross sectional views of a fist needle puncturing unit of a puncturing apparatus according to embodiment 2 of the present invention. FIG.7 shows the first needle puncturing unit before puncturing; FIG.8 shows the first needle puncturing unit when performing puncturing; and FIG.9 shows the first needle puncturing unit when being removed after puncturing.

In FIG.7, rack-and-pinion first needle puncturing unit 50 has: opening 50a; puncturing needle 50b; rack 50c configured by a toothed flat plate-like rod; and pinion 50d that has a small diameter and is integrated with a driving motor.

Puncturing needle 50b is taken out and put in opening 50a to perform puncturing. When the gear of rack 50c and the gear of pinion 50g engage with one another and a rotation force is applied to pinion 50d by the motor and so forth, this engagement of gears allows rack 50c having puncturing needle 50b to translate to linear motion, so that finally puncturing needle 50b can be moved in the backward and forward direction (vertical direction in FIG.7).

Rack & pinion (corresponding to 50c and 50d in FIG.7 to FIG.9) is a type of gear and translates a rotation force to a linear movement. When the rack & pinion obtained by combining a circular gear having a small diameter (referred to as the pinion) and the rack configured by a toothed flat plate-like rod applies a rotation force to pinion 50d, rack 50c can move straightly, i.e. in the horizontal direction (vertical direction in FIG.7 to FIG.9), up to the end of the toothed rod. In addition, spring 50e assists the pullout force when rack 50c having puncturing needle 50b is pulled out after puncturing.

Next, the operation when rack-and-pinion first needle puncturing unit 50 is employed will be described.

FIG.7 is a cross sectional view of first needle puncturing unit 50 before puncturing. In the state as shown in FIG.7, pinion 50, which is a gear having the small diameter and rack 50c, which is a toothed flat plane-like rod for linear movement having puncturing needle 50b are in the initial state. Here, puncturing needle 5b is held in the position within opening 50a of first needle puncturing unit 50 so as not to protrude from opening 50a.

When puncturing operation starts, pinion 50d coupled to a drive section such as a motor is rotated clockwise (the direction indicated by arrow 50f of FIG.7) and is transmitted to rack 50c by the gear of rack 50c, which engages with the gear of pinion 50d, so that rack 50c is moved toward opening 50a, that is, in the direction indicated by arrow 50g (downward direction in FIG.7). At this time, puncturing needle 50b mounted in rack 50c moves outside from opening section 50a.

FIG.8 is a cross sectional view of first needle puncturing unit 50 when puncturing. FIG.8 shows a state where pinion 50d is rotated by the motor in clockwise direction indicated by arrow 50f, from the state as shown in FIG.7, so that rack 50c is moved in the direction indicated by arrow 50g (downward direction in FIG.8) and reaches the position where skin is perforated, that is, skin is punctured.

FIG.9 is a cross sectional view of first needle puncturing unit 50 when puncturing needle 50a is pulled out (starts pulling) after puncturing. When the state where skin is punctured as shown in FIG.8 is created, engagement of pinion 50d and rack 50c is released by cutout part 50h of pinion 50d and spring 50e moves rack 50c in the direction indicated by arrow 50i (upward direction in FIG.9), so that puncturing needle 50b mounted in rack 50c returns to inside opening 50a again and returns to the initial state as shown in FIG.7.

In FIG.7 to FIG.9, the gear of pinion 50a having cutout part 50h where there is no gear, and this allows for limitation of the movable range by limiting the range of the gear. However, the starting position and the end position (corresponding to the standby position in FIG.7 and the puncturing position in FIG.8, respectively) can be electrically controlled by control of the motor side that rotates pinion 50d, so that cutout section 50h may not be necessarily needed and may be omitted.

In addition, as pinion 50d and the motor (not shown) that drives pinion 50d, a direct-drive integrated motor may be used. However, the direct-drive motor has not to be always required, but a belt drive motor or an idler drive motor may be applicable. Moreover, pinion 50d and the motor have not to be always integrated, but pinion 50d and the motor may be separated and coupled with one another.

As described above, since the puncturing speed can be controlled by controlling the rotational speed of the motor, the pain in puncturing can be alleviated.

(Embodiment 3)

In embodiment 3, a second needle puncturing unit is employed instead of laser puncturing unit 13 (see FIG.6) built in puncturing apparatus 1 as shown in FIG.3, as with embodiment 2. In this second needle puncturing unit, the puncturing needle is not driven by the force of a spring in the conventional way, but is driven by an electromagnetic force. By this means, the movement seed of the puncturing needle, that is, the puncturing speed can be controlled, so that puncturing with little pain can be performed.

FIG.10 to FIG.12 are cross sectional views of the second needle puncturing unit of the puncturing apparatus according to embodiment 3 of the present invention. FIG.10 shows the state of the second needle puncturing unit before puncturing; FIG.11 shows the state when puncturing is performed; and FIG.12 shows the state when the needle is pulled out after puncturing.

In FIG.10, second needle puncturing unit 51 has; opening 51a; puncturing needle 51b; first electromagnet 51 c; second electromagnet 51d; magnet (permanent magnet) 51e firmly fixed to puncturing needle 51b; and electromagnet control section 51f.

Puncturing needle 51b is taken out and put in opening 5 1 a to perform puncturing. Both first electromagnet 51c and second electromagnet 51d have therein holes penetrating them. Puncturing needle 51b is configured to slidably move through holes penetrating first electromagnet 51c and second electromagnet 51d. Electromagnet control section 51f performs puncturing, by applying electricity to first electromagnet 51c and second electromagnet 51d and making magnet 51e mounted on puncturing needle 51b generate an electromagnetic attracting force or an electromagnetic repulsion force to control to move magnet 51e mounted on puncturing needle 51b.

Next, operation when second needle puncturing unit 51 is employed will be described.

FIG. 10 is a cross sectional view of needle puncturing unit 51 adopting an electromagnetic drive system before puncturing. In the state as shown in FIG. 10, the N pole and the S pole are formed on both sides of magnet 51e (the N pole is located above magnet 51e e and the S pole is located below magnet 51 in FIG.10). In this state of magnet 51e, electromagnet control section 51f controls to first electromagnet 5 1 c to be "the S pole" and attracts magnet 51e by a magnetic force. That is, since the N pole above magnet 51e and the S pole of electromagnet 51c are attracted (moving upward in FIG.10) by the magnetic force, puncturing needle 51b on which magnet 51e is mounted also moves upward and is held. By this means, puncturing operation has been prepared.

At this time, puncturing needle 51b is located inside opening 51a that opens at one end of second needle puncturing unit 1 and is in the standby state. Since puncturing needle 51 b is evacuated into opening 51a before puncturing, so that safety is assured.

Next, when puncturing operation starts by pressing puncturing button 13j (see FIG.3), for example, electromagnetic control section 51 f switches the polarity of electromagnet 5 1 c from the S pole to the N pole to control the polarity such that second electromagnet 51d is the N pole. At this time, magnet is moved swiftly in the direction indicated by arrow 51g by the electromagnetic repulsion force of first electromagnet 51 c and the electromagnetic attractive force of second electromagnet 51d. In response to this, puncturing needle 51 b to which magnet 51e e is firmly fixed is ejected outside from opening 51a.

FIG.11 is a cross sectional view of second needle puncturing unit 51 before puncturing. In FIG.11, second needle puncturing unit 51 swiftly moves in the direction indicated by arrow 51g (downward direction of FIG.11), from the above-described state as shown in FIG.10, by the electromagnetic repulsion force between magnet 51e and first electromagnet 51c and the electromagnetic attractive force between magnet 51e and second electromagnet 51d. As a result of this, puncturing needle 51a to which magnet 51e is firmly fixed also moves swiftly in the direction indicated by arrow 51g and is ejected, and then reaches the position where skin is punctured. At this time, since magnet 51e to which puncturing needle 51b is firmly fixed is locked by second electromagnet 51d, so that the puncturing is limited in depth and excessive puncturing in depth can be prevented, so that safety is assured.

FIG.12 is a cross sectional view of second needle puncturing unit 51in the sate where the needle has been pulled out after puncturing. In FIG. 12, magnet 51e moves in the direction indicated by arrow 51h, from the state where skin is punctured as shown in FIG.11, by the electromagnetic repulsion force and the electromagnetic attractive force of each of first electromagnet 51 c and second electromagnet 51d, and returns to the standby state shown in FIG.10.

After puncturing, in order to pull put the needle, electromagnet control section 51f switches the polarity of first electromagnet 51c and second electromagnet 51d to the S pole, and then, magnet 51e moves in the direction indicated by arrow 51h (upward direction in FIG.12), by the electromagnetic attractive force between first electromagnet 51c and magnet 51e, and by the electromagnetic repulsion force between second electromagnet 51d and magnet 51e. As a result of this, puncturing needle 51b to which magnet 51e is firmly fixed also move in the direction indicated by arrow 51h and stored inside opening 51a of second needle puncturing unit 51. Then, puncturing needle 51b returns to the position in the initial standby state as shown in FIG.10.

As described above, since the puncturing speed can be controlled by controlling the current supplied to the electromagnets, so that the pain in puncturing can be alleviated.

FIG.13 is a flow chart showing operation of puncturing apparatus 11 in a case where first needle puncturing unit 50 is employed as the puncturing means of embodiment 2, or in a case where second needle puncturing unit 1 is employed as the puncturing means of embodiment 3.

Here, operation steps of the puncturing apparatus in a case first needle puncturing unit 50 or second needle puncturing unit 51 is employed, instead of laser puncturing unit 13 in FIG.14.

In step S11, control section 15f (see FIG.4, hereinafter the same) reads data of the number of remaining puncturing operations stored in memory 15d and compares it with a preset value. At this time, control section 15f determines whether the number of remaining puncturing operations is equal to or more than the predetermined value (the predetermined number of times).

When the number of remaining puncturing is less than the predetermined value, the step moves to step S12, and control section 15f makes display section 14 display a warning indication and an alarm. The warning indication in step S12 is an indication to replace battery 16 with battery 16a and to instruct to charge the battery when battery 16 is a secondary battery, which is displayed by display section 14. Here, the warning indication is to give warning to the patient and not limited to visual indication. Audio indication such as an electronic buzzer and audio response, and touch sense indication such as vibration may be applicable. After waning indication is displayed, this flow ends. In the present embodiment, when data of the number of remaining puncturing operations stored in memory 15d is less than a predetermined value, puncturing by the puncturing means (first needle puncturing unit 50 or second needle puncturing unit 1 here) in that state is forcibly stopped or terminated.

When the number of remaining puncturing operations stored in memory 15d is equal to or more than the predetermined value in step S11 described above, display section 14 displays, to the patient, that it is possible to perform puncturing along with the number of remaining puncturing operations, and the step moves to step S13 and step S14. Here, display may be performed by blinking of light-emitting diode and so forth, in addition to by display section 14.

Step S13 and step S14 are performed in parallel in the puncturing apparatus.

In step S13, first power consumption measuring section 15b measures power consumption of the puncturing step by the puncturing means (first needle puncturing unit 50 or second needle puncturing unit 51) of step S14.

In step S14, puncturing is performed using first needle puncturing unit 50 or second needle puncturing unit 51. When the patient checks that it is possible to perform puncturing and presses puncturing button 13j, the puncturing needle is ejected (where, puncturing needle 50a is ejected in a case where the puncturing unit is first needle puncturing unit 50, or puncturing needle 51a is ejected in a case where the puncturing unit is second needle puncturing unit 51), and then, the puncturing needle punctures skin 7 of the patient, so that blood 8 exudes from skin 7 and is used for blood test.

By this means, the puncturing process in above-described step S14 is completed, and as a result of this, the measurement of the power consumption for puncturing process in step S13 is completed.

In step S15, remaining battery level measuring section 15c measures the remaining battery level 16, and the measurement result of the remaining battery level is outputted to remaining puncturing calculation section 15e.

In step S16, remaining puncturing calculation section 15e calculates the number of remaining puncturing operations. Data of first power consumption measurement measured by first power consumption measuring section 15b and data of the number of remaining puncturing operations are stored in memory 15d.

In step S17, control section 15f makes display section 14 display the number of remaining puncturing operations that has been calculated by remaining puncturing calculation section 15e and ends this flow.

In addition, when the number of remaining puncturing operations is equal to or less than a predetermined value, display section 14 may give warning by a warning indication and an alarm to indicate that the number of remaining puncturing operations is small and by a response massage, in order to prompt the patient to charge and replace the secondary battery.

(Embodiment 4)

FIG.14 is a cross sectional view showing the configuration of a puncturing apparatus according to embodiment 4 of the present invention. Here, the same components as in embodiment 3 will be assigned the same reference numerals and the description of the repeated parts will be omitted.

In the present embodiment, puncturing apparatus 70 has a blood component measuring section. In addition to this, puncturing apparatus 70 has blood sensors for measuring components of blood and a negative pressure means.

In FIG.14, puncturing apparatus 70 having the blood component measuring section includes housing 12 made of resin. One side 12c of housing 12 is configured by cylindrical tubular body 12b having puncturing opening 12a. Laser puncturing unit 13 is mounted in tubular body 12b. In addition, sensor unit 72 for sampling and measuring blood is mounted to the tip of tubular body 12b. Blood sensor 73 that takes in and measures blood is placed on sensor unit 72.

Sensor unit 72 is configured by holder 72a and blood sensor 73. Holder 72a holds sensor 23 and serves to secure blood by touching skin when puncturing is performed.

Electrodes 74a to 74f of blood sensor 73 are in contact with and are connected to connectors of electrical circuit section 15 including therein the blood component measuring section, and signals from blood sensor 73 are taken into the puncturing apparatus.

In addition, holder 72a touches skin and forms a negative pressure chamber. Negative pressure means 71 has a function to apply a negative pressure to the negative pressure chamber. Negative pressure path 71a is a connection path to suck in inside the negative pressure chamber formed by holder 72a and the skin through negative pressure means 71 and puncturing opening 12a.

In addition, display section 14, puncturing button 13j and laser output setting knob 13k are mounted on the other side 12d of housing 12.

Electrical circuit section 15A controls operation of laser puncturing unit 13. Electrical circuit section 15A is housed between laser puncturing unit 13 and the other side 12d of housing 12.

In addition, buttery 13 is housed replaceably next to tubular body 12b. Both primary battery and secondary battery may be used as this battery 16. A lithium battery, an oxyride battery, an alkaline battery and so forth are used as a primary battery, meanwhile, a nickel-cadmium battery, a nickel hydride battery, lithium-ion battery and so forth are used as a secondary battery. In the present embodiment, a lithium-ion battery, which is the secondary battery is employed.

FIG. 15 is a block diagram of an electrical circuit section 15 built in puncturing apparatus 70 having the blood test measuring section and its neighborhood. Here, the same components as in FIG.4 will be assigned the same reference numerals and the description of the repeated parts will be omitted.

In FIG.15, electrical circuit section 15A has a configuration including: control section 15f configured by a microcomputer; memory 15d; timer 15h; voltage and current detecting section 15a that detects the voltage and the current of buttery 16; remaining battery level measuring section 15c that measures the remaining battery level; remaining blood test calculation section 15i; second power consumption measuring section 15j that measures the power consumption of the entire system; negative pressure means 71; blood test circuit section 77; high voltage generating circuit 81; and communicating section 82.

In addition, blood test circuit section 77 is a blood component measuring section that measures and tests components of blood, and is composed of switching circuit 75, current/voltage convertor 76, analog/digital convertor (hereinafter referred to as "A/D convertor") 78, computing section 79 and reference voltage source 80. The output of switching circuit 75 is connected to the input of current/voltage convertor 76, and the output of current/voltage convertor 76 is connected to the input of computing section 79 through A/D convertor 78. The output of computing section 79 is connected to communicating section 82 and display section 14. In addition, reference voltage source 80 is connected to switching circuit 75. Reference voltage source 80 may be a ground potential.

In addition, second power consumption measuring section 15j is a power consumption measuring section that measures power consumption of the blood component measuring section and is composed of blood test circuit power consumption measuring section 15m, negative pressure means power consumption measuring section 1511 and laser power consumption measuring section 15k that measures the power consumption of laser puncturing unit 13.

operation information from laser output setting knob 13k, puncturing button 13j and mode changing switch 13m is inputted to control section 15f of electrical circuit section 15A. In addition, remaining puncturing calculation section 15e outputs the number of remaining puncturing operations that has been calculated to display section 14 made of LCD. Here, the display section is to display the number of remaining puncturing operations, and may be configured by LED segments or organic EL.

Blood sensor 73 takes in blood exuding from skin by capillary action and analyzes components of blood after puncturing is performed. Detection electrodes 74a to 74f are formed in blood sensor 73, and detection electrodes 74a to 74f are connected to switching circuit 75 through connectors (not shown) provided in puncturing apparatus 70 body side.

Voltage and current detecting section 15a measures a voltage of battery 16 and a current flowing out from battery 16. The output of voltage and current detecting section 15a is inputted to second power consumption measuring section 15j and remaining battery level measuring section 15c.

Second power consumption measuring section 15j calculates the power consumption for one blood test operation (i.e. a series of operation including creation of negative pressure, puncturing and measurement) by integrating current value (A) flowing out from battery 16, voltage (V) of battery 16 and time (h) required for blood test. Second power consumption measuring section 15j stores data of the measured power consumption in memory 15d. Memory 15d stores data of each power consumption every time blood test is performed. The data of each power consumption, including the measurement time data is stored in memory 15d.

Blood test measuring circuit power consumption measuring section 15m measures the power consumption of blood test circuit section 77 for one blood test (measurement).

Negative pressure means power consumption measuring section 151 measures the power consumption of negative pressure means 71 for one blood test operation (puncturing and measurement).

Laser power consumption measuring section 15k measures the power consumption of laser puncturing unit 13 for one blood test operation (puncturing). via high voltage generating circuit 81.

The blood test operation refers to the operation associated with blood test, that is, a series of operation including touch with skin, creation of negative pressure, puncturing, measurement and display of the result.

Remaining battery level measuring section 15c measures the remaining battery level using the integrated circuit dedicated to the remaining battery level measurement. Remaining battery level measuring section 15c calculate the remaining battery level by multiplying the voltage value and the current value in the unloaded condition and subtracting this multiplied value from the capacity of battery 16 when battery 16 is replaced.

Remaining blood test calculation section 15i calculates the number of remaining blood tests, based on the output of remaining battery level measuring section 15c and the output of memory 15d. Specifically, remaining blood test calculation section 15i calculates by dividing the remaining battery level measured by remaining battery level measuring section 15c by the power consumption for one blood test operation measured by second power consumption measuring section 15j. As the power consumption for one blood test operation, the average of the power consumption of every blood test stored in memory 15d.

As described above, since the number of remaining blood tests is calculated using the average of the power consumption customized to each patient, it is possible to calculate accurately the number of remaining blood tests. In addition, remaining blood test calculation section 15i outputs data of the number of remaining blood tests to display section 14, according to a command from control section 15f. Display section 14 displays the number of remaining blood tests.

Accordingly, the patient can know accurately the number of remaining blood tests. By this means, for example, the patient checks the number of remaining puncturing operations before going out and charges the battery if the number of remaining puncturing operations is less than the number of times of use while going out, and consequently such a trouble that puncturing cannot perform in a place where the patient has gone, can be prevented.

The number of remaining blood tests can be calculated by dividing the remaining battery level by the fixed value or the set value of the power consumption of laser output setting knob 13k connected to control section 15f. In this case, display section 14 and electric circuit section 15 also consume electric power, so that the entire power consumption slightly fluctuates. However, since the power consumption of laser puncturing unit 13 is overwhelmingly larger than the power consumption of display section 14 and electrical circuit section 15 (about hundredfold of the power consumption of electrical circuit section 15), the remaining battery level can be calculated using the fixed value or the set value in consideration of only the power consumption of laser puncturing unit 13.

Puncturing button 13j is pressed down to activate laser puncturing unit 13, so that laser light 13h (see FIG.6) is emitted.

Laser output setting knob 13k adjusts the level of laser output in a multi-step form. In each present embodiment 1, 4 to 6, the output level of laser output setting knob 13k can set to approximately even 8 steps of laser output from 100 mJ to 600 mJ. In addition, although a knob is employed in the present embodiment, a dial may be employed, and when a touch panel is used to select from the display screen of display section 14 or when a touch panel is mounted to the screen of display section 14, input may be performed through the touch panel.

Control section 15f is configured by a microcomputer and so forth, controls puncturing operation of the entire apparatus and controls display or information of the number of remaining blood tests. Control to display the number of remaining blood tests will be described later with reference to FIG.16. Signals, from puncturing button 13j, output adjusting knob 13k, mode changing switch 13m and timer k, are inputted to control section 15f. Control section 15f outputs each control signal to the control terminal of switching circuit 75, computing section 79, high voltage generating circuit 81 that supplies a high voltage to laser puncturing unit 13, negative pressure means 71, remaining blood test calculation section 15i and communicating section 82.

Now, operation of puncturing apparatus 70 having the blood component measuring section configured as described above will be explained.

First, measurement operation of electrical circuit section 15 will be described.

First, switching circuit 75 is switched, and a detection electrode serving as a working electrode for measuring blood components is connected to current and voltage convertor 76 through the determined connector. In addition, a detection electrodes serving as a detecting electrode for detecting inflow of blood is connected to reference voltage source 80 though the determined connector.

Then, a constant voltage is applied between the detection electrode serving as a working electrode and the detection electrode serving as a detection electrode. In this state, a current flows between two detection electrodes and 42 if blood flows in the detecting section. This current is converted into a voltage by current/voltage convertor 76, and the voltage value is converted into a digital value by A/D convertor 78. Then, this digital value is outputted to computing section 79. Computing section 79 detects the fact that the blood has sufficiently flown in, based on the digital value.

When the detecting section does not detect blood even if a predetermined time period passes and when the amount of blood is not appropriate, control section 15f may make a warning means work to give an alarm and make display section 14 display the action to be taken.

Next, glucose, which is a component of blood, will be measured. To measure the glucose level, first, switching circuit 75 is switched by a command from control section 15f, and the detection electrode serving as a working electrode for measuring the glucose level is connected to current/voltage convertor 76. In addition, the detection electrode serving as a counter electrode for measuring the glucose level is connected to reference voltage source 80.

For example, while the glucose in blood and its oxidation-reduction enzyme react for a given period of time, current/voltage convertor 76 and reference voltage source 80 are turned off. Then, after a certain period of time (1 to 10 seconds) passes, a certain voltage (0.2 to 0.5 V) is applied between the detection electrode serving as the working electrode and the detection electrode serving as the counter electrode. Then, the current flown between two detection electrodes is converted into a voltage by current/voltage convertor 76. This voltage value is converted into a digital value by A/D convertor 78. This digital value is outputted to computing section 79. Computing section 79 calculates the glucose level based on this digital value.

After the glucose level is measured, a Hct (hematocrit) value will be measured. First, switching circuit 75 is switched by a command from control section 15f. Then, the detection electrode serving as a working electrode for measuring the Hct value is connected to current/voltage convertor 76 through the connector. In addition, the detection electrode serving as a counter electrode for measuring the Hct value is connected to reference voltage source 80.

Next, a certain voltage (2V to 3V) is applied between the detection electrode serving as a working electrode and the detection electrode serving as a counter electrode, by a command from control section 15f. The current flowing between two detection electrodes is converted into a voltage by current/voltage convertor 76. This voltage value is converted into a digital value by A/D convertor 78. This digital value is outputted to computing section 79. Computing section 79 calculates the Hct value based on this digital value.

With reference to a calibration curve or calibration curve table determined in advance, the glucose level is corrected using the calculated Hct value. The correction result is displayed on display section 14.

In addition, the correction result may be transmitted from communicating section 82 to an injection device for injecting insulin (used as an example of therapeutic agent). Although a radio wave may be used for this transmission, transmission is preferably performed by optical communication that does not interfere with medical equipment. When the dose of insulin to administer is automatically set based on the measurement data transmitted to the injection device, it is not necessary to set the dose of insulin to be administered by the patient, which eliminates botheration with setting. Moreover, since the dose of insulin can be set in the injection device without human work, setting error can be prevented.

Next, the operation of puncturing apparatus 70 will be described.

FIG.16 is a flow chart showing operation of puncturing apparatus 70 having a blood component measuring section and employing laser puncturing unit 13 as a puncturing means.

In step S21, control section 15f (see FIG.15, hereinafter the same) compares the number of remaining blood tests stored in memory 15d with a preset value. At this time, control section 15f determines whether the number of remaining blood tests is equal to or more than the predetermined value (the predetermined number of times).

The number of remaining blood tests refers to the number of times indicating how many times of blood tests can be performed by battery 16 currently used in puncturing apparatus 70 from now, and is the value calculated from the power consumption for one blood test as a series of blood test operation including creation of a negative pressure, puncturing and measurement.

When the number of remaining blood tests is less than the predetermined number of times, control section 15f disables laser puncturing unit 13 (see FIG.15) in step S22. Next, in step S23, control section 15f displays a warning indication. As for the warning indication in step S23, for example, display section 14 displays the indication to replace battery 16 with battery 16a and to command to charge battery 16 when battery 16 is a secondary battery. Here, the warning indication is to give warning to the patient and not limited to visual indication. Audio indication such as an electronic buzzer and audio response, and touch sense indication such as vibration may be applicable. After waning indication is displayed, this flow ends. Then, control section 15f waits to replace battery 16, or waits to complete charging when battery 16 is a secondary battery.

In the present embodiment, when the number of remaining blood tests is less than the predetermined number of times, puncturing by laser puncturing unit 13 is forcibly prohibited in that state.

When the number of remaining blood tests is equal to or more than the predetermined value in step S21 described above, display section 14 displays the number of remaining blood tests and the step moves to step S24 and step S25.

Step S24 and step S25 are performed in parallel in puncturing apparatus 70.

In step S24, second power consumption measuring section 15j starts measuring power consumption. In second power consumption measuring section 15j, laser power consumption measuring section 15k, negative pressure means power consumption measuring section 151 and blood test circuit power consumption measuring section 15m, which constitute second power consumption measuring section 15j start measuring power consumption, respectively. Laser power consumption measuring section 15k starts measuring the power consumption of laser puncturing unit 13, negative pressure means power consumption measuring section 151 starts measuring the power consumption of negative pressure means 71 and blood test circuit power consumption measuring section 15m starts measuring the power consumption of blood test circuit section 77.

In step S25, control section 15f starts charging laser puncturing unit 13 (see FIG.15).

In step S26, control section 15f starts creating a negative pressure by negative pressure means 71. This creation of a negative pressure is performed in order to swell the skin in contact with negative pressure means 71, so that the reliability of puncturing and blood sampling is improved and the pain is alleviated. The negative pressure area is a space (= storing section) formed by blood sensor 73, holder 72a (see FIG.14) and the skin of the patient from negative pressure means 71 through negative pressure path 71a and puncturing opening 12a (see FIG.14).

In step S27, control section 15f checks whether laser puncturing unit 13 has been charged.

When charging of laser puncturing unit 13 is completed, laser puncturing unit 13 performs puncturing in step S28. Here, after charging is completed, another operation may be performed such that completion of charging is displayed on the display section, and when the patient him/herself presses puncturing button 13j, laser light 13h is emitted from laser puncturing unit 13 and punctures skin 7 of the patient. By this means, blood 8 exudes from skin 7.

In step S29, blood 8 exuded by puncturing is sampled. Blood 8 exuding from swelled skin 7 is introduced, from the storing section formed in blood sensor 73, into the supply path by a negative pressure by negative pressure means 71 and capillary action, and reaches the detecting section (including the detection electrodes).

In step S30, blood test circuit section 77 measures and tests components of the blood. The blood that reaches the detecting section (including detection electrodes) of blood sensor 73 starts reacting with the test reagent placed in the vicinity of the detecting section. In response to that reaction, a current electrochemically generates. Blood test circuit section 77 measures and tests components of the blood by measuring the generated current.

In step S31, control section 15f displays the measurement result on display section 14 and stops negative pressure means 71. When negative pressure means 71 is stopped, creation of a negative pressure in the storing section is stopped.

When creation of a negative pressure is stopped in step S31 described above, power consumption measurement by second power consumption measuring section 15j that has been started in step S25 described above is completed. That is, laser power consumption measuring section 15k stops measuring the power consumption of laser puncturing unit 13, negative pressure means consumption measuring section 151 stops measuring the power consumption of negative pressure means 71 and blood test circuit power consumption measuring section 15m stops measuring the power consumption of blood test circuit section 77.

In step S32, remaining battery level measuring section 15c measures the remaining battery level 16 (see FIG.15).

In step S33, remaining blood test calculation section 15i calculates the number of remaining blood tests a. In addition, the remaining battery level measurement value of battery 16, each data measured in second power consumption measuring section 15j and data of the number of remaining blood tests are stored in memory 15d.

In addition, here, when the number of remaining blood tests is equal to or less than a predetermined value, display section 14 gives warning by a warning indication and an alarm to indicate that the number of remaining blood tests is small and by a response massage, in order to prompt the patient to charge and replace the secondary battery.

In step S34, control section 15f displays the calculated number of remaining blood tests on display 14 and ends this flow.

The operation of puncturing apparatus 70 using laser puncturing unit 13 as a puncturing means has been described.

Next, the operation of puncturing apparatus 70 using one of motor-driven first needlepuncturing unit 50 or electromagnetic-driven second needle puncturing unit 51 as a puncturing means will be described.

FIG.17 is a flowchart showing operation of puncturing apparatus 70 having a blood component measuring section and using first needle puncturing unit 50 or second needle puncturing unit 51 as the puncturing means.

In step S41, control section 15f compares the number of remaining blood tests with a predetermined value that is set in advance. At this time, control section 15f determines whether the number of remaining blood tests is equal to or more than the predetermined value.

The number of remaining blood tests refers to the number of times indicating how many more times of blood test operations can be performed by battery 16 currently used in puncturing apparatus 70 from now, and is the value calculated from the power consumption for one blood test as a series of blood test operation including creation of a negative pressure, puncturing and measurement.

When the number of remaining blood tests is less than the predetermined value, control section 15f disables first needle puncturing unit 50 or second needle puncturing unit 51 (see FIG.7 to 12) in step S42. Next, in step S43, control section 15f displays a warning indication. As for the warning indication in step S43, for example, display section 14 displays the indication to replace battery 16 with battery 16a and to command to charge battery 16 when battery 16 is a secondary battery. Here, the warning indication is to give warning to the patient and not limited to visual indication. Audio indication such as an electronic buzzer and audio response, and touch sense indication such as vibration may be applicable. After the waning indication is displayed, this flow ends. In the present embodiment, when the number of remaining blood tests is less than the predetermined value, puncturing by first needle puncturing unit 50 or second needle puncturing unit 51 built in the puncturing apparatus body in that state is forcibly prohibited.

When the number of remaining blood tests is equal to or more than the predetermined value in step S41 described above, the step moves to step S44 and step S45.

Step S44 and step S45 are performed in parallel in puncturing apparatus 70.

In step S44, second power consumption measuring section 15j starts measuring power consumption. Second power consumption measuring section 15j is composed of needle puncturing unit power consumption measuring section 15k that measures the power consumption of first needle puncturing unit 50 or second needle puncturing unit 51, negative pressure means power consumption measuring section 151 and blood test circuit power consumption measuring section 15m, and each measuring section starts measuring the power consumption. Needle puncturing unit power consumption measuring section 15k starts measuring power consumption of first needle puncturing unit 50 or second needle puncturing unit 51, negative pressure means power consumption measuring section 151 starts measuring power consumption of negative pressure means 71 and blood test circuit power consumption measuring section 15m starts measuring power consumption of blood test circuit section 77.

In step S45, control section 15f starts supplying a negative pressure by negative pressure means 71. This supply of a negative pressure is to make the skin swell in contact with negative pressure means 71, so that reliability of puncturing and blood sampling is improved and the pain is alleviated. The negative pressure area is a space (= storing section ) formed by blood sensor 73, holder 72a (see FIG.14) and the skin of the patient from negative pressure means 71 through negative pressure path 71 a and puncturing opening 12a (see FIG. 14). In step S45, a skin detecting means that detects whether skin touches puncturing apparatus 70 when supply of a negative pressure starts may be provided. It is possible to detect the output signal of this skin detecting means and to manually start the negative pressure means.

In step S46, puncturing is performed using first needle puncturing unit 50 or second needle puncturing unit 51. The patient checks that it is possible to perform puncturing and presses puncturing button 13j. Then, first needle puncturing unit 50 or second needle puncturing unit 51 ejects the puncturing needle toward skin 7 and the puncturing needle punctures skin 7 of the patient, so that blood 8 exudes from skin 7.

In step S47, blood 8 exuded by puncturing is sampled. Blood 8 exuding from swelled skin 7 is introduced, from the storing section formed in blood sensor 73, into the supply path by a negative pressure by negative pressure means 71 and capillary action, and reaches the detecting section (including the detection electrodes).

In step S48, blood test circuit section 77 measures and tests the components of the blood. The blood that reaches the detecting section (including detection electrodes) of blood sensor 73 starts reacting with test reagent placed in the vicinity of the detecting section. In response to that reaction, a current electrochemically generates. Blood test circuit section 77 measures and tests components of the blood by measuring the generated current.

In step S49, control section 15f displays the measurement result on display section 14 and stops negative pressure means 71. When negative pressure means 71 is stopped, supply of a negative pressure to the storing section is stopped.

When supply of a negative pressure is stopped in step S49 described above, power consumption measurement by second power consumption measuring section in step S44 described above is completed. That is, needle puncturing unit power consumption measuring section 15k stops measuring the power consumption of first needle puncturing unit 50 or second needle puncturing unit 51, negative pressure means consumption measuring section 151 stops measuring the power consumption of negative pressure means 71 and blood test circuit power consumption measuring section 15m stops measuring the power consumption of blood test circuit section 77.

In step S50, remaining battery level measuring section 15c measures the remaining battery level 16 (see FIG.15).

In step S51, remaining blood test calculation section 15i calculates the number of remaining blood tests a. In addition, the remaining battery level measurement value of battery 16, each data measured by second power consumption measuring section 15j and the number of remaining blood tests are stored in memory 15d.

In step S52, control section 15f displays the calculated the number of remaining blood tests on display 14 and ends this flow.

(Embodiment 5)

In embodiment 5, the configuration and the operation of the laser puncturing apparatus that displays the number of remaining puncturing operations will be described in detail further.

First, the basic concept will be described.

In the conventional embodiment as shown in FIG.1, there has been a defect that the possible number of remaining measurement (the number of remaining measurement) cannot be known because the puncturing apparatus can display only the remaining battery level. Particularly, in case of the laser puncturing apparatus, the power consumption for one measurement fluctuates because the laser setting level differs for each patient. In addition, the drive time of a suction means changes depending on hardness of skin for example, so that the power consumption fluctuates. There has been such a problem that only display of remaining battery level is not enough to know accurately the number of remaining measurement by reason of batteries and by those factors, and therefore the capacity of the battery runs short in use, so that it is not possible to perform measurement.

Therefore, as described in each embodiment 1 to 4, the number of remaining puncturing operations or the number of remaining blood tests is calculated using the power consumption and the remaining battery level when puncturing or blood test is performed, and is displayed.

In addition, according to the above-described embodiment 1 and embodiment 4, it is possible to calculate the number of remaining puncturing operations or the number of remaining blood tests by measuring the laser power consumption, which is dominant power consumption.

By this means, the patient can know the number of remaining puncturing operations and the number of remaining blood tests and can take measures to replace the battery, or charge the battery when the battery is a secondary battery before it is not possible to perform puncturing or measurement.

The inventers found that accuracy of display of the number of remaining puncturing operations can be more improved by learning individual difference among patients and making use of this individual difference for calculation of the number of remaining puncturing operations.

Computational accuracy of the number of remaining puncturing operations that can be performed is improved by gathering patient's (personal) information and learning the usage state.

The individual difference can be classified into "physical difference" and "difference in how to use".

"Physical difference" includes the thickness of skin, the hardness of skin and so forth. Meanwhile "difference in how to use" includes the frequency in use, the laser setting level, the number of retries and so forth.

[Physical difference]

First, "physical difference" will be described.

The primary parameters for individuals are the hardness of skin and the thickness of skin. There is such a relationship that the higher the laser output is, the larger the volume of hole made in skin (mainly in the depthwise direction) is. In addition, the hardness of skin of the patient correlates with low-moist skin of the patient. The higher moisture content is, the larger the volume of hole made in skin by puncturing is, for the same laser output. Therefore, it is necessary to increase the laser setting level for the patient having harder and thicker skin.

FIG.18 is a drawing showing the laser setting level measured by the inventers and its usage rate (%). As shown in FIG.18, since children tend to have softer skin than adult, many adults set the laser output higher than that for children. In addition, since women tend to have softer skin than men, it is assumed that many men set the laser out higher than that for women. As described above, the laser setting level differs greatly depending on an individual basis. On the other hand, one patient rarely changes the laser setting level that has been set.

FIG.19 is a drawing showing the relationship between the thickness of skin (mm) and the laser output (average) (mJ) required for puncturing.

In case of the current system, the laser output is about 100 mJ required for the skin having the standard thickness of 0.6 mm. The required laser output fluctuates according to the hole diameter of puncturing. The hole diameter is about 0.2 to 0.5 mm. Although the above-mentioned data is affected by the hardness of skin and so forth as described above, those numerical values can be most always obtained.

[Difference in how to use]

Next, difference in how to use will be described.

The inventers tried to estimate computational accuracy of the number of remainders based on the relationship between the frequency in use and the number of retries.

1. Frequency in use

The frequency in use refers to the frequency at which puncturing or blood test is performed. The frequency in use for patients who use often is 6 times per day and the frequency in use for patients who use not often is 3 times per week, and thus the frequency in use varies significantly depending on the level of diabetes. • Usually, the patient uses the puncturing apparatus before and after meal.

2. Retry frequency

The retry frequency refers to the frequency at which puncturing is performed more than once because puncturing is not successful. In a case where puncturing operations are performed more than once in short period of time (5 minutes only as a guide), it can be understood that the second puncturing operation is performed because the first puncturing operation is not successful, and therefore, the second puncturing operation is determined as a retry. Puncturing is performed before and after meal in general, and therefore if puncturing operations are performed more than once within 5 minutes, the puncturing must have been unsuccessful.

By those causality, it is assumed that computational accuracy of the number of remainders results in differences as shown in FIG.20.

FIG.20 is a drawing explaining the tendency of the charging cycle of a battery and computational accuracy of remainders, based on the relationship between the frequency in use and the number of retries.

i) In a case where both the frequency of retry and the frequency in use are small.

Charging cycle: maximum
Effect of the system standby power: large
Computational accuracy of the number of remainders: middle

In this condition, since the frequency in use is small, the power consumption of the battery per day is reduced. That is, a cycle at which charging is performed, or the battery is replaced is longer. By this means, the effect of the remaining battery level gradually increases due to the system standby power and self-discharge of the battery. Therefore, the error occurs in case of the number of remaining puncturing operations is calculated using only the power consumption and the remaining battery level when puncturing is performed. Accordingly, the accurate number of remaining puncturing operations can be calculated preciously by correcting the number of remaining puncturing operations with the standby power of the system and the amount of self-discharge of the battery, which are prepared in advance. In addition, since the number of retries is small, the number of puncturing operations per day is always fixed, so that the amount of the system standby power and the amount of self-discharge of the battery can be estimated accurately, and therefore the number of remaining puncturing operations can be calculated accurately.

ii) In a case where the frequency of retry is small and the frequency in use is large

Charging cycle: short
Effect of the system standby power: small
Accurate Computational accuracy of the number of reminders: high

In this condition, the frequency is high → battery is consumed fast → the charging cycle of the battery is short. Therefore, the effect of the power consumption as a result of the system standby power and self-discharge of the battery becomes small, so that the number of remaining puncturing operations can be easily obtained by simple calculation. In addition, since the number of retries is small, the number of puncturing operations per day is always fixed, so that the system standby power and the amount of self-discharge of the battery can be accurately estimated, and therefore the number of remaining puncturing operations can be accurate calculated accurately.

iii) In a case where the frequency of retries is high and the frequency in use is low

Charging cycle: long
Effect of system standby power: large
Accurate Computational accuracy of the number of remainders: low

In this condition, since the charging cycle is long as described above, the influence of the remaining battery level gradually increases as a result of the system standby power and self-discharge of the battery. In addition , since the number of retries is large, it is difficult to expect the number of times of use per day and the accuracy of the number of remaining puncturing operations degrades. In this case, in order to prevent such a situation that an expected puncturing operation cannot be performed as a result of the error, it is necessary to display larger number of remaining puncturing operations than the number of remaining puncturing operations calculated with a safety factor.

iv) In a case where both the number of retries and the frequency of use are large

Charging cycle: shortest
Effect of the system standby power: minimum
Computational accuracy of the number of remainders: middle

Since the charging cycle is short, the number of remainders is easily obtained by simple calculation. However, since the number of retries is large, it is not possible to predict how many times the apparatus will be used for some days from now, so that the computational accuracy degrades.

The estimate of calculation of the number of remainders is performed accurately and usability for user is improved, based on the above-described relationship between the frequency in use and the number of retries as follows.

The current design can afford to perform 50 times of puncturing operations by one charging. Therefore, a patient who uses frequently the puncturing apparatus needs to charge 50/6 = about 8 times, that is, in a case where the apparatus is used 6 times per day, the patient needs to charge about every week.

When it is not possible to calculate accurately, higher value than the number of times calculated actually is displayed to take the margin. That corresponds to cases of i), iii) and iv) as shown in FIG.20. For example, in order not to get the patient into trouble, such a case has to be prevented that although expecting to perform puncturing another 5 times, the patient can perform puncturing only four times. Therefore, even if actually puncturing can be still performed, the patient does not permit to perform puncturing, and in this case, the error is about 2 times at most. That is, although only 2 times among the total of 50 times are wasted, such a trouble that puncturing cannot be performed when the patent needs to perform puncturing can be prevented, and consequently the object of the present invention is sufficiently achieved. In addition, in a case where calculation is performed accurately as the case of ii) of FIG.2, more accurate calculation will be performed.

FIG.21 is a schematic diagram showing the entire configuration of the laser puncturing apparatus according to embodiment 5 of the present invention, based on the above-described basic concept. FIG.22 is a block diagram of the electrical circuit section and its neighborhood, in the above-described laser puncturing apparatus. FIG.21 and FIG.22 correspond to the puncturing apparatus according to embodiment 1 as shown in FIG.3 and FIG.4.

In FIG.21, laser puncturing apparatus 100 has a configuration including: housing 100A; and in this housing A, laser emitting device 110, which is a laser emitting means; laser output setting section 120; extra puncturing means mounting part 130; puncturing button 140; electrical circuit section 150; display section 160; and battery 170.

In FIG.22, electrical circuit section 150 has a configuration including: control section 151; voltage and current detecting section 152 that detects the current and the voltage of battery 170; power consumption measuring section 153 that measures the power consumption of the entire system; remaining battery level measuring section 154 that measures the remaining battery level; memory 155; remaining puncturing calculation section 156; laser power consumption measuring section 157 that measures the power consumption of laser emitting device 110; and timer 158. Operation information from laser output setting section 120 and puncturing button 140 is inputted to control section 151 of electrical circuit section 150. In addition, control section 151 outputs the number of remaining puncturing operations that has been calculated to display section 160 made of LCD.

Laser output setting section 120 adjusts the level of laser output in a multi-step form. In the present embodiment, laser output setting section 120 sets, for example, 8 steps of laser output (from 100 mJ to 600 mJ). The setting method includes: by an adjustable button; by a dial; inputting on the screen; and software execution.

Extra puncturing means mounting section 130 is a part for mounting an extra needle, which is an extra puncturing means that does not consume electric power. Puncturing means mounting section 130 may be mounted internally and externally. For example, a pocket that stores the extra needle may be provided internally, or a case that contains the extra needle may be mounted in the housing body.

Puncturing button 150 outputs pressing-down operation to control section 151. Control section 151 activates laser emitting device 110 by pressing down puncturing button 140.

Control section 151 is configured by a microcomputer and so forth, controls the entire operation of laser puncturing apparatus 100 including a puncturing operation and performs calculation of the number of remaining puncturing operations and display of the number of remaining puncturing operations by remaining puncturing calculation section 156. Operation of the number of remaining puncturing operations operation calculation and the remaining puncturing display will be described in detail later with reference to FIG.27.

Voltage and current detecting section 152 detects the voltage of battery 170 and the current flowing out from battery 170 and outputs the voltage and current detection result to power consumption measuring section 153 and remaining battery level measuring section 154.

Power consumption measuring section 153 calculates the power consumption by multiplying current value (A) flowing out from buttery 170 for one puncturing operation, voltage (V) of buttery 170 and time (h) required to perform puncturing.

Remaining battery level measuring section 154 is configured by a remaining battery level measurement-dedicated IC and so forth and measures the remaining battery level 170. Specifically, the remaining battery level may be calculated based on information about the voltage value in the unloaded condition, the voltage drop value in the loaded condition and the current value. Power consumption (Wh) and battery power loss (Wh) are indicated by the following formulas (1) and (2).

$$\text{Power consumption (Wh)} = \text{integral (h) of current value (A) outputted from the battery} \times \text{battery voltage value (v)} \quad ---(1)$$

$$\text{Battery power loss (Wh)} = (\text{battery voltage (V) in the unloaded condition} - \text{battery voltage (V) in the loaded condition}) \times \text{current value (A) outputted from the battery in the loaded condition} \times \text{time (h)} \quad --- (2)$$

Here, the battery power loss is generated by the battery internal resistance value. The battery internal resistance value is lager in the environment at low temperature and also larger as a result of deterioration of the battery. When this battery internal resistance is lager, the battery power loss is also lager. The battery power loss can be calculated by formula (2).

Memory 155 is configured by a nonvolatile memory, such as a EEPROM (electrically erasable programmable ROM)and a flash memory and stores power consumption data measured by power consumption measuring section 153 every time puncturing is performed.

Remaining puncturing calculation section 156 calculates the number of remaining puncturing operations, based on the output of remaining battery level measuring section 154 and the output of memory 155. In the present embodiment, remaining puncturing calculation section 156 is a remaining puncturing calculation program executed by control section 151. Remaining puncturing calculation section 156 may be configured by an electrical circuit. The method for calculating the number of remaining puncturing operations includes two types of methods, i.e. a method with a high regard for accuracy and a method with a high regard for easiness, and both the methods may be employed. In addition, when the computational accuracy can be high as with ii) of FIG.20, the method with a high regard for accuracy may be employed, meanwhile, when computational accuracy is middle or low as with i), iii) and iv) of FIG.20, the method with a high regard for easiness may be employed.

[Method with a high regard for accuracy]

Remaining puncturing calculation section 156 calculates the number of remaining puncturing operations by dividing the remaining battery level by the power consumption for one puncturing operation as shown in the following

formula (3).
$$\text{The number of remaining puncturing operations} = \frac{\text{(the remaining battery level)}}{\text{(the power consumption for one puncturing operation)}} \quad ---(3)$$

Here, the above-described power consumption includes the electric power consumed by display section 160 and electrical circuit section 150 as well as laser emitting device 110. Since the power consumption of laser emitting device 110 is the dominant power consumption, the number of remaining puncturing operations can be calculated by measuring only the power consumption of laser emitting device 110. In addition, remaining puncturing calculation section 156 may calculate the number of remaining puncturing operations, based on the average of the past power consumption stored in memory 155. The average of the past power consumption stored in memory 155 is used, so that computational accuracy can be improved.

[The method with a high regard for easiness]

The remaining puncturing calculation section 156 calculates the number of remaining puncturing operations by dividing the remaining battery level by the fixed value corresponding to the laser power set value.
$$\text{The number of remaining puncturing operations} = \frac{\text{(the remaining battery level)}}{\text{(the fixed value corresponding to the laser power set value)}} \quad ---(4)$$

Electric power is consumed by display section 160 and electrical circuit section 150 as well as laser emitting device 110. Since the power consumption of laser emitting device 110 is the dominant power consumption, the number of remaining puncturing operations may also be calculated using the fixed value corresponding to the set value of laser output setting device 120.
Laser power consumption measuring section 157 measures the power consumption of laser emitting device 110 every time puncturing is performed. This measurement result is provided to remaining puncturing calculation section 156 through control section 151 and can be used to calculate accurately the number of remaining puncturing operations of the above-described [the method with a high regard for accuracy]. In addition, the measurement result may be stored in memory 155.
Timer 158 measures a time period required to charge with a high voltage and swell skin for puncturing by laser emitting device 110. Each time period for charging with a high voltage and for swelling skin may be measured separately.
Display section 160 is composed of LCD, a driver circuit and so forth and displays puncturing information including the number of remaining puncturing operations. When the number of remaining puncturing operations is equal to or less than a predetermined value, display section 160 function as an informing means to inform and warns that fact. For example, when the number of remaining puncturing operations is equal to or less than a predetermined value, display section 160 displays in red so as to emphasize the warning. In addition, when displaying the warning, display section 160 displays an effect (warning mark, blinking and so forth) to emphasize the warning. In stead of or in combination with the information means by display 160, audio assist using an electronic buzzer or speech synthesis LSI may be applicable.
Battery 170 is a lithium-ion battery, which is a secondary battery. Here, battery 170 may be another secondary battery or a primary battery.
Now, operation of laser puncturing apparatus 100 configured as described above.
FIG.23 is a flow chart showing display operation of the number of remaining puncturing operations of laser puncturing operations apparatus 100. FIG.24 is a perspective view explaining the usage state of laser puncturing apparatus 100.
In FIG.23, First, the data of number of remaining puncturing operations stored in memory 15d is compared with a predetermined value in step S61. Here whether the number of remaining puncturing operations is equal to or more than the predetermined value or not is determined.

When the number of remaining puncturing operations is less than the predetermined value, control section 51 displays a warning indication in step S62. As for the warning indication in step S62, for example, an indication to command to charge battery 170 and an indication to puncture by the extra puncturing means mounted in extra puncturing means mounting part 130 are displayed on display section 160. Here, the warning indication is to give warning to the patient and not limited to visual indication. Audio indication such as an electronic buzzer and audio response, and touch sense indication such as vibration may be applicable. After waning indication is displayed, this flow ends. In addition, when the number of remaining puncturing operations is equal to or less than the predetermined value, puncturing by laser puncturing apparatus 100 may be prohibited.

When the number of remaining puncturing operations is equal to or more than the predetermined value in step S61, the step moves to step S63 and S64.

Step S63 and step S64 are performed in parallel in laser puncturing apparatus 100.

In step S63, power consumption measuring section 153 starts measuring power consumption.

In step S64, control section 151 starts charging laser emitting device 110.

In step S65, control section 151 checks whether laser emitting device 110 has been charged.

When charging of laser emitting device 110 is completed, puncturing is performed by laser emitting device 110 in step S66. As shown in FIG.24, skin 7 of left hand touches laser puncturing apparatus 100 held by the right hand, for example. When the patient checks the display section 160 indicating the fact that it is possible to perform puncturing, which is displayed on display section 160 and presses puncturing button 140, laser emitting device 110 emits laser light. By this means, blood 8 exudes from skin 7.

In step S67, remaining battery level measuring section 154 measures the remaining battery level 170.

In step S68, remaining puncturing calculation section 156 calculates the number of remaining puncturing operations, based on the output of remaining battery level measuring section 154 and the output of memory 155. When the method with a high regard for accuracy described above is employed, the number of remaining puncturing operations is calculated, further based on the output of laser consumption measuring section 157. In addition, memory 155 stores the measurement value of the remaining battery level 170, each data measured by laser power consumption measuring section 157 and data of the number of remaining blood tests.

In step S69, control section 151 displays the number of remaining puncturing operations that has been calculated on display section 160 and ends this flow.

As described in detail above, laser puncturing apparatus 100 includes power consumption measuring section 153 that measures the power consumption for one puncturing operation, remaining battery level measuring section 154 that measures the remaining battery level and laser power consumption measuring section 157 that measures the power consumption of laser emitting device 110, the remaining puncturing calculation section 156 calculates the number of remaining puncturing operations that can be performed based on the measured remaining battery level and power consumption, and control section 151 displays the number of remaining puncturing operations that has been calculated on display section 160. Therefore, the patient can know the number of remaining puncturing operations before puncturing is performed. By this means, for example, the patient checks the number of remaining puncturing operations before going out and charges the battery if the number of remaining puncturing operations is less than the number times of use while going out, and consequently such a trouble that puncturing cannot perform in a place where the patient has gone, can be prevented. Therefore, it is possible to administer the appropriate dose of insulin, so that degradation of the condition can be prevented.

In addition, in the present embodiment, remaining puncturing calculation section 156 calculates the number of remaining puncturing operations by dividing the remaining battery level measured by remaining battery level measuring section 154 by the power consumption for one puncturing operation by the puncturing means, which is measured by power consumption measuring section 153. The average of the power consumption of every puncturing stored in memory 155 is used as the power consumption for one puncturing operation. The power consumption for one puncturing operation differs depending on the usage condition and state of each patient. The number of remaining puncturing operations customized to each patient can be calculated by averaging the power consumption of every puncturing operation of each patient, and it is possible to calculate the number of remaining puncturing operations that is more suitable for each patient and is more accurate.

The patient can know accurately the number of remaining puncturing operations that is optimized to the patient and is reliable. By this means, such a trouble that puncturing and blood-sampling cannot be performed as a result of consumption of battery 160 can be prevented.

(Embodiment 6)

FIG.25 and FIG.26 are cross sectional views of blood test apparatus 200 according to embodiment 6 of the present invention. Here, in FIG.25 and FIG.26, blood test apparatus 200 is shown in the direction in use. The upper direction of FIG.25 and FIG.26 means the upper direction in use. FIG.27 is a perspective view of blood test apparatus 200 according to the present embodiment. FIG.28 is a perspective view of blood test apparatus 200 from the back side.

As shown in FIG.25 and FIG.26, blood test apparatus 200 has: housing 222; and in this housing, cartridge 224; holding section 225; laser emitting device 210; electrical circuit section 250; pump 228; and battery 270.

Housing 222 is made of resin and so forth, has an approximately rectangular solid shape and is composed of housing body 222a having opening 222d and cover 222b that covers opening 222d. Cover 222b is rotatably connected to housing body 222a around spindle 222c. Cover 222b rests in a state where housing is closed, that is, cover 222b covers opening 222d. In addition, cover 222b rests in the first resting position where cover 222b is open with respect to housing body 222a at about 30 degrees and rests in the second resting position where cover 222b is open with respect to housing body 222a at about 90 degrees . FIG.25 and FIG.28 show the state where cover 222 is closed, and FIG.26 shows the state where cover 222b rests in the first resting position and housing 222 is open. In addition, FIG.27 shows the state where cover 222b rests in the second resting position and housing 222 is open.

Cartridge 224 is movably mounted in housing body 22a and stacks and stores blood sensors 223. As shown in FIG.27, cartridge 224 can easily replaced by resting cover 222b in the second resting position where cover 222b is open with respect to housing body 222a at about 90 degrees.

In addition, blood sensors 23 stacked and stored in cartridge 224 are separated one by one and conveyed to holding section 225 by the conveying unit.

Holding section 225 is composed of first holder 225a provided above and second holder 225b provided below. Then, holding section 225 sandwiches and holds one blood sensor 223 separated and conveyed from cartridge 224 between first holder 225a and second holder 225b.

Laser emitting device 210, which is a puncturing means, is fixed in a position in the back of holding section 25 (the upward position in FIG.27). Laser emitting device 210 emits laser light 210h to puncture the skin of the patient in contact with second holder 225b of holding section 225. Here, focusing lens (not shown) is arranged on the tip of laser emitting device 210, and shield 210k, which is a lens protecting member, is replaceably mounted between the focusing lens and first holder 225a. Shield 210k is made of material such as glass and plastic allowing laser light to pass, and protects laser emitting device 210 and the focusing lens from evaporated materials generated during puncturing with laser light.

When laser light 210h is emitted, cover 22b rests in the first resting position at the opening angle about 30 degrees as shown in FIG.26, so that laser light 210h hits against a part of cover 222b and does not leak outside, and therefore safety is ensured.

Electrical circuit section 250 is electrically connected to sensors 223, puncturing button 240, laser emitting device 210 and pump 228. Then, electrical circuit section 250 makes laser emitting device 26 emit laser light 210h according to an electric signal indicating that puncturing button 240 has been pushed. In addition, electrical circuit section 250 analyzes components of the blood sampled in sensor 23. Moreover, electrical circuit section 250 makes pump 228, which is a negative pressure means (see FIG.29 and FIG.30), create a negative pressure at a predetermined timing.

Pump 228 creates a negative pressure in holding section 225 and cartridge 224.

Timings associated with creation of a negative pressure in holding section 225 is as follows: when skin 7 touches holding section 225, creation of a negative pressure in holding section 225 starts; and when the measurement is completed, creation of a negative pressure in holding section 225 stops. In addition, timings associated with supply of a negative pressure in cartridge 224 is as follows: when cover 222b is closed, supply of a negative pressure to cartridge 224 starts; and then after a certain time period passes, supply of a negative pressure to cartridge 224 stops. In addition, a negative pressure may be applied to inside cartridge 224 regularly at a predetermined time using timer 258 (FIG.30).

Battery 270 supplies power to laser emitting device 210, electrical circuit section 250 and pump 228.

Puncturing button 240 is provided on top surface 222e of housing 222. The patient presses puncturing button 240 to generate an electrical signal, and the generated electrical signal is outputted to electrical circuit section 250.

In addition , outlet 222q for ejecting blood sensor 223 that has been measured is provided on side surface 222g of housing 222.

Moreover, positioning convex part 222h is provided in housing 222. Positioning convex part 222h is formed by a leaf spring and fits in cartridge 224 to position cartridge 224.

In addition, display section 260 and operation button 256a (see FIG.27) are provided on front face 222n of housing 22. Display section 260 displays numerical values indicating a test result such as the property of blood (e.g. blood sugar level), and displays massages for informing the current state or a warning. In addition, display section 260 displays the number of times of blood tests. Operation button 256a operates for switching displays of display section 260, and setting and confirming registration data or a puncturing strength.

Operation lever 222f and slit hole 222m are provided on back surface 222p of housing 222. Operation lever 222f is slidably engaged with slit hole 22 (not shown) which is provided below housing body 222a. The patient slides operation lever 222f to move one sensor 223 from cartridge 224 to holding section 225. Here, when housing 222 is closed as shown in FIG.28, operation lever 222f is locked.

FIG.29 is a schematic diagram showing the entire configuration of the above-described blood test apparatus 200. FIG.30 is a block diagram of the electrical circuit section and its neighborhood, in the above-described blood test apparatus 200. Here, FIG.29 and FIG.30 correspond to block diagrams of the laser puncturing apparatus according to embodiment 5 as shown in FIG.21 and FIG.22.

In FIG.29, blood test apparatus 200 has a configuration including: housing 222; and, in this housing 222, laser emitting device 210, which is a laser emitting means; laser output setting section 220; extra puncturing means mounting part 230; puncturing button 240; electrical circuit section 250; display section 260; battery 270 and negative pressure means 280. In addition, blood sensor 223 is sandwiched by holding section 225 (FIG.25 and FIG.26), which is a sensor unit in housing 222. Detection electrodes (not shown) are formed in blood sensor 223, and these detection electrodes are connected to blood test circuit 257 through connectors and electrode 226 provided in blood test apparatus 200 body side.

In FIG.30, electrical circuit section 250 has a configuration including: control section 251 ; voltage and current detecting section 252 that detects the voltage and the current of battery 270; power consumption measuring section 253 that measures the power consumption of the entire system; remaining battery level measuring section 254 that measures the remaining battery level; memory 255; remaining blood test calculation section 256; blood test circuit section 257, timer 258; and communicating section 259. Operation information from laser output setting section 220, puncturing button 240 and operation button 256a (see FIG.27) is inputted to control section 251 of electrical circuit section 250. In addition, control section 251 outputs, to display section 260 made of LCD, the number of remaining puncturing operations that has been calculated.

Laser output setting section 220 adjusts the level of laser output in a multi-step form. The output level of laser output setting section 220 can set to approximately even 8 steps of laser output from 100 mJ to 600 mJ. In addition, although a knob is employed in the present embodiment, a dial may be employed, and when a touch panel is used to select from the display screen of display section 260 or when a touch panel is mounted to the screen of display section 260, input may be performed through the touch panel.

Blood sensor 223 takes in blood exuding from skin after puncturing by capillary action and analyzes components of the blood. Detection electrodes (not shown) of blood sensor 223 are connected to switching circuit 257b of blood test circuit 257 through connectors and electrode 226 (see FIG.29).

Here, although the present embodiment is applied to blood test apparatus 200, so that the blood sensor is adopted as the sensor, the sensor is not limited to this and any sensor is applicable provided that the sensor is an electrochemical sensor or optical sensor. That is, the blood test apparatus is applicable to measurement of blood components such as glucose, lactate acid or cholesterol levels by changing the type of sensor.

Extra puncturing means mounting section 230 is a part for mounting an extra needle, which is an extra puncturing means that does not consume electric power. The puncturing means mounting section may be mounted internally and externally. For example, a pocket that stores the extra needle may be provided internally, or a case that contains the extra needle may be mounted in the body.

Puncturing button 240 outputs pressing operation to control section 251. Control section 251 activates laser emitting device 210 by pressing puncturing button 240.

Control section 251 is configured by a microcomputer and so forth, controls the entire operation of blood test apparatus 200, including puncturing operation and blood test operation and performs calculation of the number of remaining puncturing operations and display of the number of remaining puncturing operations by remaining blood test calculation section 256. Calculation and display operation of the number of remaining puncturing operations will be described in detail later with reference to FIG.31. Signals from puncturing button 240, laser output setting section 220, operation button 256 (see FIG.27) and timer 258 are inputted to control section 251. Control section 251 outputs each control signal to a control terminal of switching circuit 257b, computing section 257e, laser emitting device 210, negative pressure means 280, remaining blood test calculation section 256 and communicating section 259.

Voltage and current detecting section 252 detects the voltage of battery 270 and the current flowing out from battery 270 and outputs the voltage and current detection result to power consumption measuring section 253 and remaining battery level measuring section 254.

Power consumption measuring section 253 calculates the power consumption by multiplying current value (A) flowing out from buttery 270 for one puncturing operation (i.e. a series of operation including creation of a negative pressure, puncturing and measurement), voltage (V) of buttery 270 and time (h) required to perform puncturing. Blood test operation refers to operation associated with blood test, that is, a series of operation including touch with skin, creation of a negative pressure, puncturing, measurement and display of the measurement result.

Power consumption measuring section 253 is a power consumption measuring section to measure the power consumption for the entire system of the blood test apparatus, and is composed of laser power consumption measuring section 253a, negative pressure means power consumption measuring section 253b and blood test circuit power consumption measuring section 253c.

Laser power consumption measuring section 253a measures the power consumption for one blood test (puncturing) operation of laser emitting device 210.

Negative pressure means power consumption measuring section 253b measures the power consumption for one blood test operation (puncturing and measurement) of negative pressure means 280.

Blood test measuring circuit power consumption measuring section 253c measures the power consumption for one blood test (measurement) of blood test circuit section 257.

Here, power consumption measuring section 253 may measure the power consumption for a series of blood test operation or may calculate the sum of each power consumption of laser emitting device 210, negative pressure means 280 and blood test circuit section 257. When the power consumption is individually calculated, the number of times of blood tests for each measurement mode. The measurement mode will be described in the paragraph of the number of blood test calculation section 256.

Remaining battery level measuring section 254 is configured by the remaining level measurement-dedicated IC and measures the remaining battery level 270. Remaining battery level measuring section 254 integrates the voltage value and the current value in the unloaded condition and subtracts the integrated value from the capacity of battery 270 when battery 270 is replaced to calculate the remaining battery level.

Memory 255 is configured by a nonvolatile memory, such as a EEPROM and a flash memory and stores each power consumption data measured by power consumption measuring section 253 every time puncturing is performed. In the present embodiment, memory 255 stores the past power consumption data to learn the number of blood tests depending on the usage state of the user. Memory 255 stores each measurement data measured by power consumption measuring section 253 and is read by the number of blood test calculation section 256.

Remaining blood test calculation section 256 calculates the number of blood tests by dividing the measured remaining battery level by the power consumption for one puncturing operation. The power consumption for one puncturing operation is measured as follows.

The power consumption of laser emitting device 210 and negative pressure means 280 is the dominant power consumption, so that the power consumption of display section 260 and electrical circuit section 260 may be disregard. However, in order to improve computational accuracy, the power consumption preferably includes the power consumption of the system as well as the power consumption of laser emitting device 210 and negative pressure means 280. In addition, remaining blood test calculation section 256 may calculate the number of remaining puncturing operations, based on the average of the past power consumption stored in memory 255. The average of the past power consumption stored in memory 255 is used, so that computational accuracy can be improved.

Since the laser power consumption depends on the laser power set value, the fixed value corresponding to the set value may be used. In addition, the blood test circuit power consumption may be the fixed value. However, since the power consumption of negative pressure means 280 varies among individuals, it is difficult to set the power consumption of negative pressure means 280 to the fixed value.

In the present embodiment, blood test apparatus 200 has a plurality of modes including: a normal test mode to execute usual puncturing, creation of a negative pressure and measurement; a negative pressure and measurement mode to execute only creation of a negative pressure and measurement; and a measurement mode to execute only measurement. Control section 251 compares the remaining battery level with a plurality of predetermined values and switches modes according to the remaining battery level. In addition, the patient may switch modes manually.

Remaining blood test calculation section 256 calculates the number of blood tests corresponding to each measurement mode.

[There is negative pressure means] •Normal test mode (laser puncturing, creation of a negative pressure and measurement)

Power consumption for one puncturing operation = laser power consumption + negative pressure means power consumption + blood test circuit power consumption •negative pressure and blood test mode (creation of negative pressure and blood test)
Power consumption for one puncturing operation =negative pressure means power consumption + blood test circuit power consumption •Blood test mode (only blood test)
Power consumption for one puncturing operation = blood test circuit power consumption

[There is not negative pressure means] •Normal test mode (laser puncturing and measurement)

Power consumption for one puncturing operation = laser power consumption + blood test circuit power consumption •Blood test mode (only blood test)
Power consumption for one puncturing operation = blood test circuit power consumption

Blood test circuit section 257 is a blood component measuring section that measures and tests components of blood and is composed of reference voltage source 257a, switching circuit 257b, current-voltage convertor 257c, A/D convertor 257d and computing section 257e. The output of switching circuit 257b is connected to the input of current/ voltage convertor 257c, and the output of current/voltage convertor 257c is connected to the input of computing section 257e through A/D convertor 257d. The output of computing section 257e is connected to communicating section 259 and display section 260. In addition, reference voltage source 257a is connected to switching circuit 257b. Reference voltage source may be a ground potential.

Timer 258 measures a time period required to charge with a high voltage and swell skin for puncturing by laser emitting device 210. Each time period for charging with a high voltage and for swelling skin may be measured separately.

Display section 260 is composed of LCD, driver circuit and so forth and displays puncturing information including the number of remaining puncturing operations. When the number of remaining puncturing operations is equal to or less than a predetermined value, display section 260 function as an informing means to inform and warns that fact. For example, when the number of remaining blood tests is equal to or less than a predetermined value, display section 260 displays in red so as to emphasize the warning. In addition, when displaying the warning, display section 160 displays an effect (warning mark, blinking and so forth) to emphasize the warning. In the present embodiment, blood test apparatus 200 has a plurality of measurement modes, that is, a one-step measurement mode, a negative pressure and measurement mode and a measurement mode. When the measurement mode is switched, display section 260 displays the number of times of measurement that can be performed, for each mode. In stead of or in combination with the information means by display 260, audio assist using an electronic buzzer or speech synthesis LSI may be applicable.

In the present embodiment, the lithium-ion battery, which is a secondary battery is employed. Here, battery 170 may be another secondary battery or a primary battery.

Negative pressure means 280 sucks in blood from skin using a negative pressure. Although the present embodiment includes negative pressure means 280, the negative pressure has not to always be included and a case where there is no negative pressure means 280 (only by massaging the skin and so forth) may be applicable.

Now, operation of blood test apparatus 200 configured as described above will be explained.

First, measurement operation of electrical circuit section 250 will be described.

First, switching circuit 257b is switched, and the detection electrode serving as a working electrode is connected to current/voltage convertor 257c through the determined connector. In addition, the detection electrode serving as a detecting electrode for detecting inflow of blood is reference voltage source 257a through the determined connector.

Then, a constant voltage is applied between the detection electrode serving as a working electrode and the detection electrode serving as a detecting electrode. In this state, a current flows between two detection electrodes if blood flows in the detecting section. This current is converted into a voltage by current/voltage convertor 257c and the voltage value is converted into a digital value by A/D convertor 257d. Then, this digital value is outputted to computing section 257e. Computing section 257e detects that the blood has sufficiently flown in, based on the digital value.

When the detecting section does not detect blood even if a predetermined time period passes and when the amount of blood is not appropriate, the control section may make a warning means work to give an alarm and make display section 260 display the action to be taken.

Next, glucose, which is a component of blood, will be measured. To measure the glucose level, first, switching circuit 257b is switched by a command from control section 251, and the detection electrode 41 serving as a working electrode for measuring the glucose level is connected to current/voltage convertor 257c through the connector. In addition, the detection electrode serving as a counter electrode for measuring the glucose level is connected to reference voltage source 257a through the connector.

For example, while the glucose in blood and its oxidation-reduction enzyme react for a given period of time, current/voltage convertor 257c and reference voltage source 257a are turned off. Then, after the certain period of time (1 to 10 seconds) passes, a certain voltage (0.2 to 0.5 V) is applied between the detection electrode serving as a working electrode and the detection electrode serving as a counter electrode by a command from control section 251. Then the current flown between two detection electrodes is converted into a voltage by current/voltage convertor 257. This voltage value is converted into a digital value by A/D convertor 257d. This digital value is outputted to computing section 257e. Computing section 257e calculates a glucose level based on this digital value.

Next, after the glucose level is measured, Hct value is measured. First, switching circuit 257b is switched by a command from control section 251. The detection electrode serving as a working electrode for measuring the Hct value is connected to current/voltage convertor 257c through the connector. In addition, the detection electrode serving as a counter electrode for measuring the Hct value is connected to reference voltage source 257a through the connector.

Next, a constant voltage (2V to 3V) is applied between the detection electrode serving as a working electrode and the detection electrode serving as a detecting electrode by a command from control section 251. The current flowing between two detection electrodes is converted into a voltage by current/voltage convertor 257c. This voltage value is converted into a digital value by A/D convertor 257d. This digital value is outputted to computing section 257e. Computing section 257e calculates the Hct value based on this digital value.

With reference to a calibration curve or calibration curve table determined in advance, the glucose level is corrected using the calculated Hct value.

In addition, the correction result may be transmitted from communicating section 259 to an injection device for injecting insulin. Although a radio wave may be used for this transmission, transmission is preferably performed by optical communication that does not interfere with medical equipment. When the dose of insulin to administer is automatically set based on the measurement data transmitted to the injection device, it is not necessary to set the dose of insulin to be administered by the patient, which eliminates botheration with setting. Moreover, since the dose of insulin can be set in the injection device without human work, setting error can be prevented.

Next, operation of blood test apparatus 200 to display the number of times of blood tests will be described.

FIG.31 is a flow chart showing operation of blood test apparatus 200 to display the number of times of blood tests.

Control section 251 compares the remaining battery level with a plurality of thresholds and switches modes according to the remaining battery level. In addition, the blood test apparatus may accept mode switching by operating operation button 256a by the patient.

In step 571, first, control section 251 judges whether the number of remaining blood tests a is equal to or more than a predetermined value. Control section 251 also judges whether the remaining battery level 270 measured by remaining battery level measuring section 254 is equal to or more than the first set value. This first set value is a threshold for determining whether "normal test mode" is executed by battery 270 that is currently used in blood test apparatus 200. When the remaining battery level is lower than the first set value, the step moves to step S72.

In step S72, control section 251 judges whether the number of remaining blood tests b is equal to or more than a predetermined value. Control section 251 also judges whether the remaining battery level is equal to or more that the second set value smaller than the first set value. This second set value is a threshold for determining that "negative pressure and measuring mode" or "measuring mode" is executed by battery 270 that is currently used in blood test apparatus 200.

[Normal test mode]

When the number of remaining blood tests a is equal to or more than the predetermined value and the remaining battery level is equal to or more than the first set value, the mode moves to "normal test mode". Here, although control section 251 both has judged the number of remaining blood tests a and has judged the remaining battery level, control section 251 may judge only one of them.

In "normal test mode", step S101 and step S102 are processed in parallel in blood test apparatus 200.

In step S101, power consumption measuring section 253 starts measuring power consumption. That is, laser power consumption measuring section 253a starts measuring the power consumption for one test operation (puncturing) of laser emitting device 210. In addition, negative pressure means power consumption measuring section 253b starts measuring the power consumption for one test operation (puncturing and measurement) of negative pressure means 280. Moreover, blood test measuring circuit power consumption measuring section 253c starts measuring the power consumption for one test operation (measurement) of blood test circuit section 257.

In step S102, control section 251 starts charging laser emitting device 210.

In step S103, control section 251 starts creating a negative pressure by negative pressure means 280. This creation of a negative pressure to swell skin to improve the reliability of puncturing and blood-sampling and alleviate the pain. Negative pressure means 280 creates a negative pressure in a space (= storing section) formed by blood sensor 223, holding section 225, cartridge 224 (see FIG.25) and the skin of the patient via the negative pressure path and the puncturing opening (not shown).

In step S104, control section 251 checks whether laser emitting device 210 has been charged.

When charging of laser emitting device 210 is completed, puncturing is performed by laser emitting device 210 in step S105. When the patient presses puncturing button 240 according to the instruction "it is possible to perform puncturing" displayed on display section 260, laser light 210h is emitted from laser emitting device 210 and punctures skin 7 of the patient.

Then skin 7 is punctured, so that blood 8 exudes from skin 7.

In step S106, blood 8 exuding by puncturing is sampled. Blood 8 exuding from swelled skin 7 is introduced, from the storing section formed in blood sensor 73, into the supply path by a negative pressure created by negative pressure means 71 and capillary action, and reaches the detecting section (including the detection electrodes).

In step S107, blood test circuit section 257 measures and tests components of the blood. The blood that reaches the detecting section (including detection electrodes) of blood sensor 223 starts reacting with test reagent placed in the vicinity of the detecting section. In response to that reaction, a current electrochemically generates. Blood test circuit section 257 measures and tests components of the blood by measuring the generated current.

In step S108, control section 251 displays the measurement result on display section 260 and stops creating negative pressure means 280. Negative pressure means 280 stops creating a negative pressure, and consequently supply of a negative pressure to storing section is stopped.

When creation of a negative pressure is stopped in step S108 described above, power consumption measurement by second power consumption measuring section 253 that has been started in step S101 described above is completed. That is, laser power consumption measuring section 253a stops measuring the power consumption of laser emitting device 210, negative pressure consumption measuring section 253b stops measuring the power consumption of negative pressure means 280 and blood test circuit power consumption section 253c stops measuring the power consumption of blood test circuit section 257.

In step S109, remaining battery level measuring section 254 measures the remaining battery level 27.

In step S110, remaining blood test calculation section 256 calculates the number of remaining blood tests a. In addition, the measurement value of the remaining battery level 270, each data measured by power consumption measuring section 253 and data of the number of remaining blood tests are stored in memory 225.

In step S111, control section 251 displays the number of remaining blood tests a that has been calculated on display 260 and exits "normal test mode" to end this flow.

[Negative pressure and measurement mode]

When the remaining battery level is equal to or more than the second set value in the above-described step S72, the mode moves to "negative pressure and measurement mode". Here, although control section 251 has judged both the number of remaining blood tests b and the remaining battery level, control section 251 may judge only one of them.

In step S121, control section 251 disables laser emitting device 210. In addition, control section 251 displays the fact that laser emitting device 210 is disabled on display section 260.

In step S 122, the patient operates the extra needle to puncture skin and the step moves to step S 123 and step 124.

Step S123 and step S124 are processed in parallel in blood test apparatus 200.

In step S123, power consumption measuring section 253 starts measuring power consumption. That is, negative pressure means power consumption measuring section 253b starts measuring the power consumption of negative pressure means 280 for one test operation (puncturing and measurement), and blood test measuring circuit power consumption measuring section 253c starts measuring the power consumption of blood test circuit section 257 for one test operation (measurement).

In step S124, control section 251 starts creating a negative pressure by negative pressure means 280.

In step 125, components of blood 8, which exudes from skin 7 by puncturing with the extra needle, is measured and tested.

In step S126, control section 251 displays the measurement result on display 260 and stops negative pressure means 280.

When the negative pressure means is stopped in the above-described step S126, power consumption measurement by power consumption measuring section 253, which has been started in the above-described step S124 is completed. That is, negative pressure means power consumption measuring section 253b stops measuring the power consumption of negative pressure means 280, blood test measuring circuit power consumption measuring section 253c stops measuring the power consumption of blood test circuit section 257.

In step S127, remaining battery level measuring section 254 measures the remaining battery level 270.

In step S128, remaining blood test calculation section 256 calculates the number of remaining blood tests b. In addition, memory 255 stores the measurement value of the remaining battery level 270, each data measured by power consumption measuring section 253 and data of the number of remaining blood tests data.

In step S129, control section 251 displays, on display section 260, the number of remaining blood tests b that has been calculated and exits "negative pressure and measurement mode" to end this flow.

[Measurement mode]

When the remaining battery level is less than the second set value, the step moves to "measurement mode".

In step S131, control section 251 disables laser emitting device 210. In addition, control section 251 displays the fact that laser emitting device 210 is disabled on display section 260.

In step S132, control section 251 stops negative pressure means 280.

In step S133, the patient operates the extra needle to puncture skin and the step moves to step S134 and step 135.

S134 and step S135 are processed in parallel in blood test apparatus 200.

In step S134, power consumption measuring section 253 starts measuring power consumption. That is, blood test measuring circuit power consumption measuring section 253c starts measuring the power consumption for one test operation (measurement) of blood test circuit section 257.

In step 135, components of blood 8, which exudes from skin 7 by puncturing with the extra needle, is measured and tested.

In step S136, control section 251 displays the measurement result on display 260 and stops negative pressure means 280.

When the negative pressure means is stopped in the above-described step S136, power consumption measurement by power consumption measuring section 253, which has been started in the above-described step S124, that is, measurement of the power consumption of blood test circuit section 275 by blood test measuring circuit power consumption measuring section 253 is completed.

In step S137, remaining battery level measuring section 254 measures the remaining battery level 270.

In step S138, remaining blood test calculation section 256 calculates the number of remaining blood tests c. In addition, memory 255 stores the measurement value of the remaining battery level 270, each data measured by power consumption measuring section 253 and data of the number of remaining blood tests.

In step S139, control section 251 displays, on display section 260, the number of remaining blood tests c that has been calculated and exits "measurement mode" to end this flow.

As described above, blood test apparatus 200 has power consumption measuring section 253 that measures the power consumption for one puncturing operation, remaining battery level measuring section 254 that measures the remaining battery level, blood test circuit section 257 that tests blood and negative pressure means 280, remaining blood test calculation section 256 calculates the number of remaining blood tests that can be performed based on the measured remaining battery level and power consumption, and control section 151 displays, on display section 260, the number of remaining blood tests that has been calculated. Therefore, the patient can know the number of remaining blood tests before performing puncturing. By this means, for example, the patient checks the number of remaining puncturing operations before going out and charges the battery if the number of remaining puncturing operations is less than the number of times of use while going out, and consequently such a trouble that puncturing cannot perform in a place where the patient has gone, can be prevented. Therefore, it is possible to administer the appropriate dose of insulin, so that degradation of the condition can be prevented.

In addition, in the present embodiment, power consumption measuring section 253 has laser power consumption measuring section 253a that measures the power consumption for one test operation (puncturing) of laser emitting device 210, negative pressure means power consumption measuring section 253b that measures the power consumption for one test operation (puncturing and measuring) of negative pressure means 280 and blood test measuring circuit power consumption measuring section 253c that measures the power consumption for one test operation (measurement) of blood test circuit section 257, so that the number of remaining blood tests can be more calculated accurately.

Moreover, in the present embodiment, blood test apparatus 200 has a plurality of measurement modes, that is, the normal test mode, the negative pressure and measurement mode, and the measurement mode. Control section 251 compares the remaining battery level with a plurality of thresholds and switches the modes according to the remaining battery level. For example, control section 251 controls such that when the remaining battery level measured by remaining battery level measuring section 254 is equal to or less than a first threshold, control section 251 prohibits laser emitting device 210 from performing puncturing and moves to a first mode where blood test is performed only by blood test circuit section 257; when the measured remaining battery level is equal to or less than a second threshold, control section 251 prohibits laser emitting device 210 from performing puncturing and moves to a second mode where blood test is performed only by negative pressure means 280 and blood test circuit section 257; and when the measured remaining battery level is less than a third threshold, control section 251 prohibits laser emitting device 210 from performing puncturing and prohibits negative pressure means 280 from operating and moves to a third mode where blood test is performed only by blood test circuit 257.

The above description is illustration of the preferred embodiments of the present invention and the scope of the invention is not limited to this.

Moreover, although the names "puncturing apparatus" and "blood test apparatus" are used in the present embodiment for convenience of explanation, the name of the apparatus may be a "test apparatus", and the name of the method can be a "method for controlling a puncturing apparatus" and so forth.

Moreover, for each component constituting the puncturing apparatus and the blood test apparatus, such as the kind of cartridge, the number and the connection method thereof are not limited.

The puncturing method described above can be provided by a program to function this puncturing method. This program is stored in a computer-readable recording medium.

The present invention claims priority based on Japanese Patent Application No.2007-205284, filed on August 7, 2007.

Industrial Applicability

The puncturing apparatus, the blood test apparatus and the puncturing method are applicable to a blood test apparatus that punctures kin by a puncturing means such as a laser puncturing device, samples blood exuding from the skin and analyzes components of the blood.

In addition, the laser puncturing device according to the present invention can display the number of remaining puncturing operations, and therefore is applicable to a portable medical equipment that consumes a large amount of power.

**Claims**

1. A puncturing apparatus comprising:

   a housing;
   a puncturing section that is provided in the housing and that punctures skin;
   a battery that supplies power to the puncturing section;
   a remaining battery level measuring section that measures a remaining level of the battery;
   a power consumption measuring section that measures electric power consumed by the battery for puncturing operation of the puncturing section;
   a remaining puncturing calculation section that calculates a number of remaining puncturing operations that can be performed by the puncturing section, based on the remaining battery level and the power consumption that have been measured; and
   an informing section that informs the number of remaining puncturing operations calculated by the remaining puncturing calculation section.

2. The puncturing apparatus according to claim 1, wherein the puncturing section is a laser emitting device that punctures the skin with laser light.

3. The puncturing apparatus according to claim 1, wherein the puncturing section is a needle puncturing device that punctures the skin with a puncture needle.

4. The puncturing apparatus according to claim 1, wherein the power consumption measuring section measures electric power consumed by one puncturing operation.

5. The puncturing apparatus according to claim 1, further comprising a storage section that stores a measurement result of the power consumption,
   wherein the remaining puncturing calculation section calculates the number of remaining puncturing operations, based on an average of a measurement result of a past power consumption stored in the storage section.

6. The puncturing apparatus according to claim 1, further comprising a personal information acquiring section that acquires personal information including usage history of a test subject,
   wherein the remaining puncturing calculation section calculates the number of remaining puncturing operations, based on information acquired by the personal information acquiring section.

7. The puncturing apparatus according to claim 1, wherein the informing section informs the number of remaining puncturing operations calculated by the remaining puncturing calculation section, using at least one of display, sound and voice.

8. The puncturing apparatus according to claim 1, wherein when the number of remaining puncturing operations calculated by the remaining puncturing calculation section is equal to or less than a predetermined value, the informing section informs that fact.

9. The puncturing apparatus according to claim 1, wherein the informing section has a plurality of informing modes including display, sound, voice and these combination, its informing order and informs in a changed informing mode when the number of remaining puncturing operations is equal to or less than a predetermined value.

10. The puncturing apparatus according to claim 1, further comprising a control section that performs control to prohibit the puncturing section from puncturing when the number of remaining puncturing operations calculated by the remaining puncturing calculation section is equal to or less than a predetermined value.

11. The puncturing apparatus according to claim 1, further comprising a negative pressure section that applies a negative pressure to the skin,
    wherein the power consumption measuring section further measures the power consumption consumed by the negative pressure section.

12. A blood test apparatus comprising:

   a puncturing section that punctures skin with laser light;
   a blood test section that tests blood;
   a battery that supplies power to the puncturing section and the blood test section;a remaining battery level measuring section that measures a remaining level of the battery;
   a power consumption measuring section that measures electric power consumed by the battery for puncturing operation of the puncturing section;
   a remaining blood test calculation section that calculates a number of remaining blood tests that can be performed by the blood test section, based on the remaining battery level and the power consumption that have been measured; and
   an informing section that informs the number of remaining blood tests calculated by the remaining blood test calculation section.

13. The blood test apparatus according to claim 12, wherein the power consumption measuring section measures the power consumption consumed by one puncturing and test operation.

14. The blood test apparatus according to claim 13, further comprising a storage section that stores a measurement result of the power consumption,
   wherein the remaining blood test calculation section calculates a number of remaining puncturing operations, based on an average of a measurement result of a past power consumption stored in the storage section.

15. The blood test apparatus according to claim 12, further comprising a personal information acquiring section that acquires personal information including usage history of a test subject,
   wherein the remaining blood test calculation section calculates the number of remaining blood tests, based on information acquired by the personal information acquiring section.

16. The blood test apparatus according to claim 12, wherein the informing section informs the number of remaining blood tests calculated by the remaining blood test calculation section, using at least one of display, sound and voice.

17. The blood test apparatus according to claim 12, further comprising a control section that controls to prohibit the puncturing section from puncturing when the number of remaining blood tests calculated by the remaining blood test calculation section is equal to or less than a predetermined value.

18. The blood test apparatus according to claim 12, further comprising a negative pressure section that applies a negative pressure to the skin,
   wherein the power consumption measuring section further measures the power consumption consumed by the negative pressure section.

19. A puncturing method comprising:

   measuring a remaining battery level that supplies power to a puncturing section;
   measuring electric power consumed by the battery for puncturing operation of the puncturing section;
   calculating a number of remaining puncturing operations that can be performed by the puncturing section, based on the remaining battery level and the power consumption that have been measured; and
   informing the number of remaining puncturing operations that has been calculated.

20. A puncturing method comprising:

   measuring a remaining battery level that supplies power to a negative pressure section and a puncturing section;
   measuring electric power consumed by the battery for negative pressure creating operation of the negative pressure section and for puncturing operation of the puncturing section;
   calculating a number of remaining puncturing operations that can be performed by the puncturing section, based on the remaining battery level and the power consumption that have been measured; and
   informing the number of remaining puncturing operations that has been calculated.

**21.** A puncturing method comprising:

measuring a remaining battery level that supplies power to a blood test section that measures components of blood, a negative pressure section that creates a negative pressure and a puncturing section;
measuring electric power consumed by the battery for negative pressure creating operation of the negative pressure section, for puncturing operation of the puncturing section and for operation of the blood test section;
calculating a number of remaining blood tests that can be performed by the blood test section, based on the remaining battery level and the power consumption that have been measured
informing the number of remaining blood tests that has been calculated.

**Patentansprüche**

**1.** Einstechvorrichtung, die umfasst:

ein Gehäuse,
einen Einstechabschnitt, der in dem Gehäuse vorgesehen ist und in die Haut einsticht,
eine Batterie, die den Einstechabschnitt mit Strom versorgt,
einen Verbleibender-Batteriepegel-Messabschnitt, der einen verbleibenden Pegel der Batterie misst,
einen Stromverbrauchs-Messabschnitt, der den durch die Batterie für den Einstechvorgang des Einstechabschnitts verbrauchten Strom misst,
einen Verbleibende-Einstiche-Berechnungsabschnitt, der eine Anzahl von verbleibenden Einstechvorgängen, die durch den Einstechabschnitt durchgeführt werden können, auf der Basis des gemessenen verbleibenden Batteriepegels und des gemessenen Stromverbrauchs berechnet, und
einen Angabeabschnitt, der die durch den Verbleibende-Einstiche-Berechnungsabschnitt berechnete Anzahl von verbleibenden Einstechvorgängen angibt.

**2.** Einstechvorrichtung nach Anspruch 1, wobei der Einstechabschnitt eine Laseremissionseinrichtung ist, die mit Laserlicht in die Haut einsticht.

**3.** Einstechvorrichtung nach Anspruch 1, wobei der Einstechabschnitt eine Nadeleinstecheinrichtung ist, die mit einer Stechnadel in die Haut einsticht.

**4.** Einstechvorrichtung nach Anspruch 1, wobei der Stromverbrauchs-Messabschnitt den durch einen einzelnen Einstechvorgang verbrauchten Strom misst.

**5.** Einstechvorrichtung nach Anspruch 1, die weiterhin einen Speicherabschnitt umfasst, der ein Messergebnis zu dem Stromverbrauch speichert,
wobei der Verbleibende-Einstiche-Berechnungsabschnitt die Anzahl von verbleibenden Einstechvorgängen auf der Basis eines Durchschnitts eines in dem Speicherabschnitt gespeicherten Messergebnisses zu einem vergangenen Stromverbrauch berechnet.

**6.** Einstechvorrichtung nach Anspruch 1, die weiterhin einen Persönliche-Informationen-Erfassungsabschnitt umfasst, der persönliche Informationen einschließlich einer Nutzungsgeschichte einer Testperson erfasst,
wobei der Verbleibende-Einstiche-Berechnungsabschnitt die Anzahl von verbleibenden Einstechvorgängen auf der Basis von durch den Persönliche-Informationen-Erfassungsabschnitt erfassten Informationen berechnet.

**7.** Einstechvorrichtung nach Anspruch 1, wobei der Angabeabschnitt die durch den Verbleibende-Einstiche-Berechnungsabschnitt berechnete Anzahl von verbleibenden Einstechvorgängen unter Verwendung von Bild, Klang und/oder Sprache angibt.

**8.** Einstechvorrichtung nach Anspruch 1, wobei, wenn die durch den Verbleibende-Einstiche-Berechnungsabschnitt berechnete Anzahl von verbleibenden Einstechvorgängen gleich oder kleiner als ein vorbestimmter Wert ist, der Angabeabschnitt diese Tatsache angibt.

**9.** Einstechvorrichtung nach Anspruch 1, wobei der Angabeabschnitt eine Vielzahl von Informationsmodi einschließlich von Bild, Klang und Sprache oder einer Kombination oder Abfolge aus denselben aufweist und in einem anderen Informationsmodus betrieben wird, wenn die Anzahl von verbleibenden Einstechvorgängen gleich oder kleiner als ein vorbestimmter Wert ist.

10. Einstechvorrichtung nach Anspruch 1, der weiterhin einen Steuerabschnitt umfasst, der eine Steuerung durchführt, um ein Einstechen des Einstechabschnitts zu verhindern, wenn die durch den Verbleibende-Einstiche-Berechnungsabschnitt berechnete Anzahl von verbleibenden Einstechvorgängen gleich oder kleiner als ein vorbestimmter Wert ist.

11. Einstechvorrichtung nach Anspruch 1, die weiterhin einen Unterdruckabschnitt umfasst, der einen Unterdruck auf die Haut ausübt,
wobei der Stromverbrauch-Messabschnitt weiterhin den durch den Unterdruckabschnitt verbrauchten Strom misst.

12. Bluttestvorrichtung, die umfasst:

einen Einstechabschnitt, der mit Laserlicht in die Haut einsticht,
einen Bluttestabschnitt, der Blut testet,
eine Batterie, die den Einstechabschnitt und den Bluttestabschnitt mit Strom versorgt,
einen Verbleibender-Batteriepegel-Messabschnitt, der einen verbleibenden Pegel der Batterie misst,
einen Stromverbrauch-Messabschnitt, der den durch die Batterie für den Einstechvorgang des Einstechabschnitts verbrauchten Strom misst,
einen Verbleibende-Bluttests-Berechnungsabschnitt der die Anzahl von verbleibenden Bluttests, die durch den Bluttestabschnitt durchgeführt werden können, auf der Basis des gemessenen verbleibenden Batteriepegels und des gemessenen Stromverbrauchs berechnet, und
einen Angabeabschnitt, der die durch den Verbleibende-Bluttests-Berechnungsabschnitt berechnete Anzahl von verbleibenden Bluttests angibt.

13. Bluttestvorrichtung nach Anspruch 12, wobei der Stromverbrauch-Messabschnitt den durch einen einzelnen Einstech- und Testvorgang verbrauchten Strom misst.

14. Bluttestvorrichtung nach Anspruch 13, die weiterhin einen Speicherabschnitt umfasst, der ein Messergebnis zu dem Stromverbrauch speichert,
wobei der Verbleibende-Bluttests-Berechnungsabschnitt eine Anzahl von verbleibenden Einstechvorgängen auf der Basis des Durchschnitts eines in dem Speicherabschnitt gespeicherten Messergebnisses zu einem vergangenen Stromverbrauch berechnet.

15. Bluttestvorrichtung nach Anspruch 12, die weiterhin einen Persönliche-Informationen-Erfassungsabschnitt umfasst, der persönliche Informationen einschließlich einer Nutzungsgeschichte einer Testperson erfasst,
wobei der Verbleibende-Bluttests-Berechnungsabschnitt die Anzahl der verbleibenden Bluttests auf der Basis von durch den Persönliche-Informationen-Erfassungsabschnitt erfassten Informationen berechnet.

16. Bluttestvorrichtung nach Anspruch 12, wobei der Angabeabschnitt die durch den Verbleibende-Bluttests-Berechnungsabschnitt berechnete Anzahl von verbleibenden Bluttests unter Verwendung von Bild, Klang und/oder Sprache angibt.

17. Bluttestvorrichtung nach Anspruch 12, die weiterhin einen Steuerabschnitt umfasst, der eine Steuerung durchführt, um ein Einstechen des Einstechabschnitts zu verhindern, wenn die durch den Verbleibende-Bluttests-Berechnungsabschnitt berechnete Anzahl von verbleibenden Bluttests gleich oder kleiner als ein vorbestimmter Wert ist.

18. Bluttestvorrichtung nach Anspruch 12, die weiterhin einen Unterdruckabschnitt umfasst, der einen Unterdruck auf die Haut ausübt,
wobei der Stromverbrauch-Messabschnitt weiterhin den durch den Unterdruckabschnitt verbrauchten Strom misst.

19. Einstechverfahren, das umfasst:

Messen eines verbleibenden Batteriepegels, der einen Einstechabschnitt mit Strom versorgt,
Messen der durch die Batterie für den Einstechvorgang des Einstechabschnitts verbrauchten Stroms,
Berechnen einer Anzahl von verbleibenden Einstechvorgängen, die durch den Einstechabschnitt durchgeführt werden können, auf der Basis des gemessenen verbleibenden Batteriepegels und des gemessenen Stromverbrauchs, und

Angeben der berechneten Anzahl von verbleibenden Einstechvorgängen.

20. Einstechverfahren, das umfasst:

Messen eines verbleibenden Batteriepegels, der einen Unterdruckabschnitt und einen Einstechabschnitt mit Strom versorgt,
Messen des durch die Batterie für den Unterdruckerzeugungsvorgang des Unterdruckabschnitts und für den Einstechvorgang des Einstechabschnitts verbrauchten Stroms,
Berechnen einer Anzahl von verbleibenden Einstechvorgängen, die durch den Einstechabschnitt durchgeführt werden können, auf der Basis des gemessenen verbleibenden Batteriepegels und des gemessenen Stromverbrauchs, und
Angeben der berechneten Anzahl von verbleibenden Einstechvorgängen.

21. Einstechverfahren, das umfasst:

Messen eines verbleibenden Batteriepegels, der einen Bluttestabschnitt, der Komponenten des Bluts misst, einen Unterdruckabschnitt, der einen Unterdruck erzeugt, und einen Einstechabschnitt mit Strom versorgt,
Messen der durch die Batterie für dem Unterdruckerzeugungsvorgang des Unterdruckabschnitts, für den Einstechvorgang des Einstechabschnitts und für den Betrieb des Bluttestabschnitts verbrauchten Stroms,
Berechnen einer Anzahl von verbleibenden Bluttests, die durch den Bluttestabschnitt durchgeführt werden können, auf der Basis des gemessenen verbleibenden Batteriepegels und des gemessenen Stromverbrauchs,
Angeben der berechneten Anzahl von verbleibenden Bluttests.

**Revendications**

1. Appareil de ponction, comprenant :

un boîtier ;
une section de ponction agencée dans le boîtier et qui ponctionne la peau ;
une batterie qui alimente la section de ponction ;
une section de mesure du niveau de batterie restant qui mesure un niveau restant de la batterie ;
une section de mesure de consommation d'énergie qui mesure l'énergie électrique consommée par la batterie pour l'opération de ponction de la section de ponction ;
une section de calcul des ponctions restantes qui calcule un nombre d'opérations de ponction restantes qui peuvent être effectuées par la section de ponction en fonction du niveau de batterie restant et de la consommation d'énergie qui ont été mesurés ; et
une section d'information qui informe du nombre d'opérations de ponction restantes calculé par la section de calcul des ponctions restantes.

2. Appareil de ponction selon la revendication 1, dans lequel la section de ponction est un dispositif émettant un laser qui ponctionne la peau avec de la lumière laser.

3. Appareil de ponction selon la revendication 1, dans lequel la section de ponction est un dispositif de ponction à aiguille qui ponctionne la peau avec une aiguille de ponction.

4. Appareil de ponction selon la revendication 1, dans lequel la section de mesure de consommation d'énergie mesure l'énergie électrique consommée par une opération de ponction.

5. Appareil de ponction selon la revendication 1, comprenant en outre une section de stockage qui stocke un résultat de mesure de la consommation d'énergie,
dans lequel la section de calcul des ponctions restantes calcule le nombre d'opérations de ponction restantes en fonction d'une moyenne de résultats de mesure d'une consommation d'énergie passée stockée dans la section de stockage.

6. Appareil de ponction selon la revendication 1, comprenant en outre une section d'acquisition d'information personnelle qui acquiert de l'information personnelle comprenant un historique d'utilisation d'un sujet d'essai,
dans lequel la section de calcul des ponctions restantes calcule le nombre d'opérations de ponction restantes en fonction d'information acquise par la section d'acquisition d'information personnelle.

7. Appareil de ponction selon la revendication 1, dans lequel la section d'information informe du nombre d'opérations de ponction restantes calculé par la section de calcul des ponctions restantes, en utilisant au moins un mode parmi les modes d'information à affichage, sonore et vocal.

8. Appareil de ponction selon la revendication 1, dans lequel, lorsque le nombre d'opérations de ponction restantes calculé par la section de calcul des ponctions restantes est inférieur ou égal à une valeur prédéterminée, la section d'information informe de ce fait.

9. Appareil de ponction selon la revendication 1, dans lequel la section d'information comporte une pluralité de modes d'information, y compris un mode d'information à affichage, sonore, vocal ou combinant ceux-ci, envoie une commande d'information et informe dans un mode d'information changé lorsque le nombre d'opérations de ponction restantes est inférieur ou égal à une valeur prédéterminée.

10. Appareil de ponction selon la revendication 1, comprenant en outre une section de commande qui réalise une commande pour empêcher que la section de ponction n'effectue une ponction lorsque le nombre d'opérations de ponction restantes calculé par la section de calcul des ponctions restantes est inférieur ou égal à une valeur prédéterminée.

11. Appareil de ponction selon la revendication 1, comprenant en outre une section de pression négative qui applique une pression négative sur la peau,
dans lequel la section de mesure de consommation d'énergie mesure en outre l'énergie consommée par la section de pression négative.

12. Appareil d'analyse du sang, comprenant :

une section de ponction qui ponctionne la peau avec de la lumière laser ;
une section d'analyse du sang qui analyse le sang ;
une batterie qui alimente la section de ponction et la section d'analyse du sang ;
une section de mesure du niveau de batterie restant qui mesure un niveau restant de la batterie ;
une section de mesure de consommation d'énergie qui mesure l'énergie électrique consommée par la batterie pour l'opération de ponction de la section de ponction ;
une section de calcul des analyses de sang restantes qui calcule un nombre d'analyses de sang restantes qui peuvent être effectuées par la section d'analyse du sang, en fonction du niveau de batterie restant et de la consommation d'énergie qui ont été mesurés ; et
une section d'information qui informe du nombre d'analyses de sang restantes calculé par la section de calcul des analyses de sang restantes.

13. Appareil d'analyse du sang selon la revendication 12, dans lequel la section de mesure de consommation d'énergie mesure l'énergie consommée par une opération de ponction et d'analyse.

14. Appareil d'analyse du sang selon la revendication 13, comprenant en outre une section de stockage qui stocke un résultat de mesure de la consommation d'énergie,
dans lequel la section de calcul des analyses de sang restantes calcule un nombre d'opérations de ponction restantes en fonction d'une moyenne de résultats de mesure d'une consommation d'énergie passée stockée dans la section de stockage.

15. Appareil d'analyse du sang selon la revendication 12, comprenant en outre une section d'acquisition d'information personnelle qui acquiert de l'information personnelle comprenant un historique d'utilisation d'un sujet d'essai,
dans lequel la section de calcul des analyses de sang restantes calcule le nombre d'analyses de sang restantes en fonction d'information acquise par la section d'acquisition d'information personnelle.

16. Appareil d'analyse du sang selon la revendication 12, dans lequel la section d'information informe du nombre d'analyses de sang restantes calculé par la section de calcul des analyses de sang restantes, en utilisant au moins un mode parmi les modes d'information à affichage, sonore et vocal.

17. Appareil d'analyse du sang selon la revendication 12, comprenant en outre une section de commande qui réalise une commande pour empêcher que la section de ponction n'effectue une ponction lorsque le nombre d'analyses de sang restantes calculé par la section de calcul des analyses de sang restantes est inférieur ou égal à une valeur prédéterminée.

18. Appareil d'analyse du sang selon la revendication 12, comprenant en outre une section de pression négative qui applique une pression négative sur la peau,
dans lequel la section de mesure de consommation d'énergie mesure en outre l'énergie consommée par la section de pression négative.

19. Procédé de ponction, comprenant :

la mesure d'un niveau de batterie restant d'une batterie qui alimente une section de ponction ;
la mesure de l'énergie électrique consommée par la batterie pour l'opération de ponction de la section de ponction ;
le calcul d'un nombre d'opérations de ponction restantes qui peuvent être effectuées par la section de ponction, en fonction du niveau de batterie restant et de la consommation d'énergie qui ont été mesurés ; et
l'information du nombre d'opérations de ponction restantes qui a été calculé.

20. Procédé de ponction, comprenant :

la mesure d'un niveau de batterie restant d'une batterie qui alimente une section de pression négative et une section de ponction ;
la mesure de l'énergie électrique consommée par la batterie pour l'opération de production d'une pression négative de la section de pression négative et pour l'opération de ponction de la section de ponction ;
le calcul d'un nombre d'opérations de ponction restantes qui peuvent être effectuées par la section de ponction, en fonction du niveau de batterie restant et de la consommation d'énergie qui ont été mesurés ; et
l'information du nombre d'opérations de ponction restantes qui a été calculé.

21. Procédé de ponction, comprenant :

la mesure d'un niveau de batterie restant d'une batterie qui alimente une section d'analyse du sang qui mesure des composants du sang, une section de pression négative qui crée une pression négative et une section de ponction ;
la mesure de l'énergie électrique consommée par la batterie pour l'opération de production d'une pression négative de la section de pression négative, pour l'opération de ponction de la section de ponction et pour le fonctionnement de la section d'analyse du sang ;
le calcul d'un nombre d'analyses de sang restantes qui peuvent être effectuées par la section d'analyse du sang, en fonction du niveau de batterie restant et de la consommation d'énergie qui ont été mesurés,
et l'information du nombre d'analyses de sang restantes qui a été calculé.

FIG.1

FIG.2

FIG.3

FIG.4

- 13j PUNCTURING BUTTON
- 13k LASER OUTPUT SETTING KNOB
- 13 LASER PUNCTURING UNIT
- 15 (enclosure)
- 15f CONTROL SECTION
- 15h TIMER
- 15e REMAINING PUNCTURING CALCULATION SECTION
- 15g LASER POWER CONSUMPTION MEASURING SECTION
- 15d MEMORY
- 15c REMAINING BATTERY LEVEL MEASURING SECTION
- 15b FIRST POWER CONSUMPTION MEASURING SECTION
- 15a VOLTAGE AND CURRENT DETECTING SECTION
- 14 DISPLAY SECTION
- 16 BATTERY

START

S1
THE NUMBER
OF REMAINING PUNCTURING
IS EQUAL TO OR MORE THAN
PREDETERMINED
VALUE?

NO

YES

S3
START MEASURING POWER

S4
START LASER CHARGING

S5
CHARGING
HAS BEEN COMPLETED?

NO

YES

S2
DISPLAY WARNING

S6
PUNCTURE

S7
TERMINATE MEASURING POWER

MEASURE LASER POWER CONSUMPTION

S8
MEASURE REMAINING BATTERY LEVEL

S9
CALCULATE THE NUMBER
OF REMAINING PUNCTURING

S10
DISPLAY THE NUMBER
OF REMAINING PUNCTURING

END

FIG.5

FIG.6

1: BEFORE PUNCTURING

50

50e

50c

50g

50d

50f

50b

50a

FIG.7

2: DURING PUNCTURING

FIG.8

3: DURING REMOVING

FIG.9

1: BEFORE PUNCTURING

51

51 c

51 e

N
S

51 f

51 g

51 d

51 b

51 a

FIG.10

2: DURING PUNCTURING

51

51c

51g

51e

51d

51f

N
S

51b

51a

FIG.11

3: DURING REMOVING

51

51c

51e

N
S

51h

51f

51d

51b

51a

FIG.12

FIG.13

FIG.14

FIG.15

FIG.16

```
                    ┌─────────────────┐
                    │      START      │
                    └────────┬────────┘
                             │
                             ▼
                    ╱─────────────────╲                S41
                  ╱   THE NUMBER        ╲ ──────────────────►  NO
                 ⟨  OF REMAINING TEST a IS EQUAL TO ⟩
                  ╲  OR MORE THAN PREDETERMINED    ╱
                    ╲      VALUE?     ╱                         │
                      ╲─────────────╱                          ▼
                             │ YES                    ┌──────────────────┐  S42
                             │                        │  PROHIBIT LASER  │
                             ▼                        │  PUNCTURING UNIT │
              ┌──────────────────────────────┐  S45   │  FROM PUNCTURING │
         ┌───▶│ START CREATING NEGATIVE PRESSURE │     └─────────┬────────┘
         │    └──────────────┬───────────────┘                   │
         │                   │ YES                               ▼
         │                   ▼                        ┌──────────────────┐  S43
         │          ┌─────────────────┐  S46          │  DISPLAY WARNING │
         │          │    PUNCTURE     │               └─────────┬────────┘
         │          └────────┬────────┘                         │
  ┌──────────────┐           │                                  │
  │ MEASURE POWER│           ▼                                  │
  │ CONSUMPTION  │  ┌─────────────────┐  S47                    │
  │·PUNCTURING UNIT│ │  SAMPLE BLOOD   │                        │
  │·NEGATIVE PRESSURE│└───────┬────────┘                        │
  │  MEANS       │           │                                  │
  │·BLOOD TEST SECTION│       ▼                                 │
  └──────┬───────┘  ┌─────────────────┐  S48                    │
    S44  │          │     MEASURE     │                         │
         │          └────────┬────────┘                         │
         │                   │                                  │
         │                   ▼                                  │
         │    ┌──────────────────────────────┐  S49             │
         └───▶│ STOP CREATING NEGATIVE PRESSURE │                │
              └──────────────┬───────────────┘                  │
                             │                                  │
                             ▼                                  │
              ┌──────────────────────────────┐  S50             │
              │ MEASURE REMAINING BATTERY LEVEL │                │
              └──────────────┬───────────────┘                  │
                             │                                  │
                             ▼                                  │
              ┌──────────────────────────────┐  S51             │
              │  CALCULATE THE NUMBER OF      │                  │
              │     REMAINING TEST a          │                  │
              └──────────────┬───────────────┘                  │
                             │                                  │
                             ▼                                  │
              ┌──────────────────────────────┐  S52             │
              │ DISPLAY THE NUMBER OF REMAINING TEST a │         │
              └──────────────┬───────────────┘                  │
                             │◄─────────────────────────────────┘
                             ▼
                    ┌─────────────────┐
                    │       END       │
                    └─────────────────┘
```

FIG.17

FIG.18

FIG.19

FREQUENCY IN USE

( ii )

· CHARGING CYCLE : SHORT

· EFFECT OF SYSTEM STANDBY POWER
  : SMALL

· COMPUTATIONAL ACCURACY : HIGH

( iv )

· CHARGING CYCLE : MINIMUM

· EFFECT OF SYSTEM STANDBY POWER
  : MINIMUM

· COMPUTATIONAL ACCURACY : LOW

( i )

· CHARGING CYCLE : MAXIMUM

· EFFECT OF SYSTEM STANDBY POWER
  : LARGE

· COMPUTATIONAL ACCURACY: MIDDLE

( iii )

· CHARGING CYCLE :LONG

· EFFECT OF SYSTEM STANDBY POWER
  :MINIMUM

· COMPUTATIONAL ACCURACY: MIDDLE

FREQUENCY OF RETRY

FIG.20

100

120

100A

140

150

ELECTRICAL CIRCUIT SECTION

154

REMAINING BATTERY LEVEL
MEASURING SECTION

153

POWER CONSUMPTION
MEASURING SECTION

155

MEMORY

156

REMAINING PUNCTURING
CALCULATION SECTION

130

170

BATTERY

160

DISPLAY SECTION

LASER
EMITTING
DEVICE

110

FIG.21

FIG.22

EP 2 174 592 B1

START

S61

THE NUMBER
OF REMAINING PUNCTURING IS EQUAL TO
OR MORE THAN PREDETERMINED
VALUE?

NO

YES

S64

START LASER CHARGING

S65

LASER
CHARGING HAS BEEN
COMPLETED?

NO

S63

MEASURE POWER
CONSUMPTION

YES

S66

PUNCTURE

S62

DISPLAY
WARNING

S67

MEASURE REMAINING
BATTERY LEVEL

S68

CALCULATE THE NUMBER
OF REMAINING PUNCTURING

S69

DISPLAY THE NUMBER
OF REMAINING PUNCTURING

END

FIG.23

FIG.24

FIG.25

**FIG.26**

FIG.27

240

222e

200

222a

222p

222b

222c

222f

FIG.28

220

222

240

200

230

ELECTRICAL CIRCUIT SECTION

250

REMAINING BATTERY LEVEL
MEASURING SECTION

254

POWER CONSUMPTION
MEASURING SECTION

253

MEMORY

255

REMAINING AMOUNT OF BLOOD TEST
CALCULATION SECTION

256

BLOOD TEST CIRCUIT

257

280

NEGATIVE
PRESSURE MEANS

270

BATTERY

260

DISPLAY
SECTION

LASER
EMITTING
DEVICE

210

226

223

225

FIG.29

200

250

ELECTRICAL CIRCUIT SECTION

257

BLOOD TEST CIRCUIT SECTION 257

257b 257c 257d 257e

SWITCHING CIRCUIT

257a REFERENCE VOLTAGE

CURRENT/ VOLTAGE CONVERTER

A/D CONVERTER

COMPUTING SECTION

223

225

210 LASER EMITTING DEVICE

NEGATIVE PRESSURE MEANS

MEMORY 280

REMAINING BLOOD TEST CALCULATION SECTION

255 256

251 258

TIMER

CONTROL SECTION

259

COMMUNICATING SECTION

DISPLAY SECTION

260

LASER POWER CONSUMPTION MEASURING SECTION 253a

NEGATIVE PRESSURE MEANS POWER CONSUMPTION MEASURING SECTION 253b

BLOOD TEST CIRCUIT POWER CONSUMPTION MEASURING SECTION

REMAINING BATTERY LEVEL MEASURING SECTION

253c POWER CONSUMPTION MEASURING SECTION

254

253

VOLTAGE AND CURRENT DETECTING SECTION 252

BATTERY

270

PUNCTURING BUTTON

240

LASER OUTPUT SETTING SECTION

220

FIG.30

FIG.31

**EP 2 174 592 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004533866 PCT
- KR 200420950 Y1
- JP 2007205284 A